(19) **Europäisches Patentamt / European Patent Office / Office européen des brevets**

(11) **EP 4 059 450 A1**

(12) **EUROPEAN PATENT APPLICATION**

(43) Date of publication:
**21.09.2022 Bulletin 2022/38**

(21) Application number: **22156402.4**

(22) Date of filing: **07.03.2014**

(51) International Patent Classification (IPC):
**A61B 17/20** (2006.01)    **A61M 37/00** (2006.01)
**A61K 9/00** (2006.01)

(52) Cooperative Patent Classification (CPC):
**A61B 17/205; A61K 9/0021;** A61M 2037/0023;
A61M 2037/0046; A61M 2037/0053; Y02A 50/30

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361800543 P**

(62) Document number(s) of the earlier application(s) in accordance with Art. 76 EPC:
**14713727.7 / 2 967 597**

(71) Applicant: **Corium, Inc.**
**Boston, MA 02210 (US)**

(72) Inventors:
• **CHEN, Guohua**
**Sunnyvale, CA 94086 (US)**

• **DING, Zhongli**
**Sunnyvale, CA 94087 (US)**
• **GHARTEY-TAGOE, Esi**
**San Jose, CA 95134 (US)**
• **SINGH, Parminder**
**Union City, CA 94587 (US)**

(74) Representative: **D Young & Co LLP**
**120 Holborn**
**London EC1N 2DY (GB)**

Remarks:
•This application was filed on 11.02.2022 as a divisional application to the application mentioned under INID code 62.
•Claims filed after the date of receipt of the divional application (Rule 68(4) EPC).

(54) **MICROSTRUCTURE ARRAY FOR DELIVERY OF ACTIVE AGENTS**

(57)    Provided herein is a microstructure array comprising a plurality of dissolving microstructures such as microprojections attached to a base. The plurality of microstructures comprise an active agent in a biocompatible and water-soluble matrix, where the water-soluble matrix preferably comprises a polysaccharide polymer and a sugar alcohol, and the base typically comprises a non-water soluble matrix. The plurality of microstructures, upon penetration of the subject's skin, undergo dissolution to deliver the active agent. Also provided are related microstructure formulations, in dried and liquid form, methods for preparing the above-described microstructure arrays, and methods for administering an active agent by application of a microstructure array as provided herein to a subject's skin, among other features.

FIG. 1

**Description**

CROSS-REFERENCE TO RELATED APPLICATIONS

This application claims the benefit of U.S. Provisional Application No. 61/800,543, filed March 15, 2013, which is incorporated herein by reference in its entirety.

TECHNICAL FIELD

The disclosure relates generally to a delivery system, composition, and method for transdermally administering a therapeutic agent using an array of microstructures, and related features thereof.

BACKGROUND

Arrays of microneedles were proposed as a way of administering drugs through the skin in the 1970s. Microneedle or microstructure arrays can facilitate the passage of drugs through or into human skin and other biological membranes in circumstances where ordinary transdermal administration is inadequate. Microstructure arrays can also be used to sample fluids found in the vicinity of a biological membrane such as interstitial fluid, which is then tested for the presence of biomarkers.

In recent years, microstructure arrays have been prepared in a manner that makes financially feasible their widespread use. U.S. Pat. No. 6,451,240 discloses illustrative methods of manufacturing microneedle arrays. If the arrays are sufficiently inexpensive, they can be provided as disposable devices. A disposable device is preferable to a reusable device, since the integrity of the device is not compromised due to prior use, and the potential need of resterilizing the device after each use, and maintaining the device under controlled storage conditions is eliminated. Moreover, microstructure arrays are advantageous for use in developing countries, since the need for needles and refrigeration is eliminated.

Despite much initial work on fabricating microneedle arrays using silicon or metals, there are significant advantages to polymeric rather than metal or silicon-based arrays. Methods of manufacturing polymeric microneedle arrays are described in U.S. Patent No. 6,451,240, while arrays prepared primarily of biodegradable polymers have also been described. See, e.g., U.S. Pat. No. 6,945,952 and U.S. Published Patent Application Nos. 2002/0082543 and 2005/0197308. A detailed description of the fabrication of an illustrative microneedle array made of polyglycolic acid (PGA) is found in Jung-Hwan Park et al., "Biodegradable polymer microneedles: Fabrication, mechanics, and transdermal drug delivery," J. of Controlled Release, 104:51-66 (2005). Vaccine delivery via microneedle arrays is described, for example, in U.S. Patent Publication No. 2009/0155330, which is incorporated herein by reference. Dissolving microprojection arrays are also described therein.

Microneedle-assisted transdermal delivery of therapeutic agents is a fairly recent technological development. There exists a current need for improved formulations and microprojection arrays for effectively delivering active agents, small molecule drugs and larger molecules such as proteins and peptides, via the skin, while providing good formulation stability (including maintenance of active agent potency) upon manufacturing and storage, and during administration, to thereby conveniently deliver a therapeutically and/or immunogenically effective amount of active agent without the associated discomfort, inconvenience, or chemical instability of traditional liquid-based, needle-based methods.

BRIEF SUMMARY

The following aspects and embodiments described and illustrated below are meant to be exemplary and illustrative, and are no way intended to be limiting in scope.

In a first aspect, provided herein is an array of microstructures comprising an approximately planar base and a plurality of microstructures, where the array comprises at least one active agent.

More particularly, provided is a microstructure array comprising an approximately planar base and a plurality of dissolving microstructures, each microstructure having an attachment point to the base and a distal tip to penetrate a subject's skin, wherein (i) the plurality of microstructures comprises an active agent in a biocompatible and water-soluble matrix, the biocompatible and water-soluble matrix comprising a polysaccharide polymer and a sugar alcohol, and (ii) the base comprises a biocompatible, non-water soluble polymer matrix, wherein the microstructures, or at least a portion thereof, upon penetration of the subject's skin, undergo dissolution to thereby deliver the active agent.

In one embodiment of the microstructure array, the polysaccharide is a glucan or a chemically-modified glucan.

In yet another embodiment, the polysaccharide is an alpha-glucan or a chemically modified alpha-glucan.

In yet a more particular embodiment, the polysaccharide is a dextran or a chemically-modified starch such as carboxymethyl (CM) starch or a hydroxyalkylstarch. Exemplary hydroxyalkyl starches include hydroxyethylstarch (HES) or hydroxypropylstarch (HPS). In yet a further embodiment, the chemically-modified starch has a degree of substitution ranging from about 0.80 to 0.40.

In an embodiment directed to the sugar alcohol, the sugar alcohol is selected from the group consisting of glycerol, xylitol, mannitol, sorbitol, galactitol, lactitol, erythritol, glycerol, and maltitol. In yet another embodiment, the sugar alcohol is sorbitol.

In a specific and preferred embodiment of a microstructure array, the polysaccharide is dextran and the sugar alcohol is sorbitol.

In yet another specific and preferred embodiment of a microstructure array, the polysaccharide is hydroxyethylstarch and the sugar alcohol is sorbitol.

In yet an additional embodiment, the biocompatible and water-soluble matrix further comprises one or more excipients or adjuvants. In a related embodiment, the one or more excipients is a surfactant.

In a further embodiment, a microstructure array as provided herein is characterized by an active agent-comprising biocompatible and water-soluble matrix, which, when dissolved in aqueous buffer at an active agent concentration ranging from about 0.1% to about 7% by weight, is further characterized by stability of the active agent for at least 7 days at 5°C. That is to say, the liquid formulation, which upon drying, results in formation of the active agent-comprising biocompatible and water-soluble matrix, exhibits a solution phase stability with respect to the active agent as set forth above.

In a more specific embodiment of the solid microstructure array, the plurality of microstructures comprises from about 1 - 15 weight % (solids) active agent, from about 40-75 weight % (solids) polysaccharide, and from about 25-40 weight % (solids) sugar alcohol.

In a second aspect, provided herein is a liquid formulation suitable for forming a plurality of dissolving microstructures, where the liquid formulation comprises an active agent, a polysaccharide, and a sugar alcohol in a buffer. In a particular embodiment related to the foregoing, the liquid formulation comprises from about 3-20% by weight polysaccharide, from about 1-15% by weight sugar alcohol, and from about 0.05-5% by weight active agent.

In one or more embodiments related to the second aspect (i.e., the liquid formulation which upon drying forms the biocompatible, water-soluble, active agent-comprising matrix), the polysaccharide, sugar alcohol, and additional optional excipients are as described above in embodiments related to the first aspect.

In yet another and third aspect, provided herein is a liquid formulation as described above in dried form.

In a fourth aspect, provided is a method of making a microstructure array. The method comprises the following steps: (i) providing a liquid formulation comprising an active agent, a polysaccharide and a sugar alcohol in a buffer, wherein the liquid formulation comprises from about 3-20% by weight polysaccharide, from about 1-15% by weight sugar alcohol, and from about 0.05-5% by weight active agent; (ii) dispensing the liquid formulation from (i) onto a mold having an array of microstructure cavities and filling the microstructure cavities to form a formulation-filled mold, (iii) drying the formulation-filled mold, (iv) placing a backing layer on the dried mold from (iii), whereby the backing layer forms a base having an attachment point to each of the microstructure cavities to provide a molded microstructure array, and (v) removing the microstructure array from (iv) from the mold.

In an embodiment related to the above, the method further comprises affixing the backing layer to a backing substrate. Exemplary backing substrates include, e.g., a breathable non-woven pressure sensitive adhesive and an ultraviolet-cured adhesive in a polycarbonate film.

In a further embodiment related to the method, following the dispensing step, excess liquid formulation is removed from the surface of the mold.

In a fifth aspect, provided is a method of transdermally administering an active agent to a mammalian subject, comprising inserting into the skin of the subject a microstructure array having the features set forth herein.

In an embodiment related to the method of administering, the microstructure array comprises an approximately planar base and a plurality of dissolving microstructures, each microstructure having an attachment point to the base and a distal tip to penetrate a subject's skin. The plurality of microstructures comprises an active agent in a biocompatible and water-soluble matrix, where the biocompatible and water-soluble matrix comprises a polysaccharide polymer and a sugar alcohol, and the base comprises a biocompatible non-water soluble polymer matrix, wherein the microstructures, upon penetration of the subject's skin, undergo dissolution to thereby deliver the active agent.

Additional embodiments of the present microstructures, arrays, methods, and the like, will be apparent from the following description, drawings, examples, and claims. As can be appreciated from the foregoing and following description, each and every feature described herein, and each and every combination of two or more of such features, is included within the scope of the present disclosure provided that the features included in such a combination are not mutually inconsistent. In addition, any feature or combination of features may be specifically excluded from any embodiment of the present invention.

Additional aspects and advantages of the present invention are set forth in the following description and claims, particularly when considered in conjunction with the accompanying examples and drawings.

BRIEF DESCRIPTION OF DRAWINGS

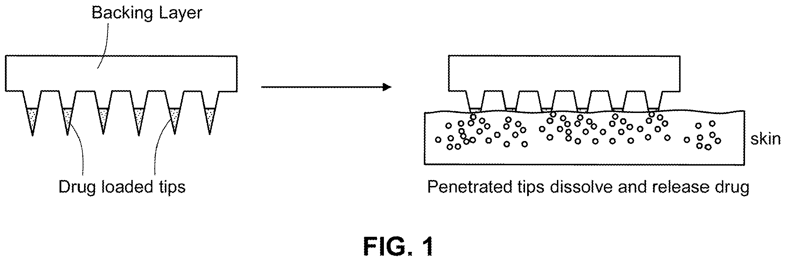

FIG. 1 is a schematic illustration of an exemplary microstructure array as provided herein both before (left hand side) and after application to the skin (right hand side). The microstructures are capable of penetrating the stratum corneum barrier layer of the skin to facilitate the delivery of a therapeutic agent such as an active agent. The microstructure array shown is composed of a biodegradable tip or microstructure portion (indicated as a drug loaded tip) and a backing (also referred to herein as a base) layer. The distal portion of the microstructures contains dried active agent in a water-soluble and biocompatible matrix. The backing layer that connects and supports the tip portion is typically composed of a non-water soluble and biocompatible matrix. When inserted into the skin, the active agent-loaded tips dissolve and release the active agent into the skin.

DETAILED DESCRIPTION

Various aspects of the microstructure array, active agent formulations, and related methods will be described more fully hereinafter. Such aspects may, however, be embodied in many different forms and should not be construed as limited to the embodiments set forth herein; rather, these embodiments are provided so that this disclosure will be thorough and complete, and will fully convey its scope to those skilled in the art.

The practice of the present disclosure will employ, unless otherwise indicated, conventional methods of chemistry, biochemistry, and pharmacology, within the skill of the art. Such techniques are explained fully in the literature. See, *e.g.;* A.L. Lehninger, Biochemistry (Worth Publishers, Inc., current addition); Morrison and Boyd, *Organic Chemistry* (Allyn and Bacon, Inc., current addition); J. March, Advanced Organic Chemistry (McGraw Hill, current addition); Remington: The Science and Practice of Pharmacy, A. Gennaro, Ed., 20th Ed.; Goodman & Gilman The Pharmacological Basis of Therapeutics, J. Griffith Hardman, L. L. Limbird, A. Gilman, 10th Ed.

Where a range of values is provided, it is intended that each intervening value between the upper and lower limit of that range and any other stated or intervening value in that stated range is encompassed within the disclosure. For example, if a range of 1 $\mu$m to 8 $\mu$m is stated, it is intended that 2 $\mu$m, 3 $\mu$m, 4 $\mu$m, 5 $\mu$m, 6 $\mu$m, and 7 $\mu$m are also explicitly disclosed, as well as the range of values greater than or equal to 1 $\mu$m and the range of values less than or equal to 8 $\mu$m.

Definitions

As used in this specification, the singular forms "a," "an," and "the" include plural referents unless the context clearly dictates otherwise. Thus, for example, reference to a "polymer" includes a single polymer as well as two or more of the same or different polymers, reference to an "excipient" includes a single excipient as well as two or more of the same or different excipients, and the like.

In describing and claiming the present invention, the following terminology will be used in accordance with the definitions described below.

"Biodegradable" refers to natural or synthetic materials that degrade enzymatically, non-enzymatically or both to produce biocompatible and/or toxicologically safe by-products which may be eliminated by normal metabolic pathways.

"Hydrophobic polymer" as used herein refers to polymers that are insoluble or poorly soluble in aqueous solvents. "Hydrophilic polymer" as used herein refers to polymers that are soluble or substantially soluble in aqueous solvents.

The terms "microprotrusion", "microprojection", "microstructure" or "microneedle" are used interchangeably herein to refer to elements adapted to penetrate or pierce at least a portion of the stratum corneum or other biological membranes. For example, illustrative microstructures may include, in addition to those described herein, microblades as described in U.S. Patent No. 6,219,574, edged microneedles as described in U.S. Patent No. 6,652,478, and microprotrusions as described in U.S. Patent Publication No. U.S. 2008/0269685 and U.S. 2009/0155330.

"Optional" or "optionally" means that the subsequently described circumstance may or may not occur, so that the description includes instances where the circumstance occurs and instances where it does not.

"Substantially" or "essentially" means nearly totally or completely, for instance, 90% or greater of some given quantity.

"Transdermal" refers to the delivery of an agent into and/or through the skin for local and/or systemic therapy. The same inventive principles apply to administration through other biological membranes such as those which line the interior of the mouth, gastro-intestinal tract, blood-brain barrier, or other body tissues or organs or biological membranes which are exposed or accessible during surgery or during procedures such as laparoscopy or endoscopy.

A material that is "water-soluble" may be defined as soluble or substantially soluble in aqueous solvents, such that the material dissolves into, within or below the skin or other membrane which is substantially aqueous in nature.

Overview

The present disclosure is directed, at least in part, to the discovery of a preferred combination of components for use in preparing a biocompatible and water-soluble matrix comprising an active agent, e.g., for use in a microstructure array for transdermally administering the active agent. More specifically, the inventors have developed a combination of a polysaccharide polymer and a sugar alcohol for forming a biocompatible and water-soluble matrix comprising an active agent in dried form. The sugar alcohol, such as sorbitol, possesses a dual role in the instant formulations. More specifically, the sugar alcohol functions to both stabilize the active agent components (proteins, peptides, polynucleotides, small molecule drugs, etc.), particularly in the dried state, and additionally functions to plasticize the polysaccharide component. The combination of a polysaccharide and a sugar alcohol, when used to form a biocompatible and water-soluble matrix for use in a microstructure array, and in particular, for use in the microstructures themselves, provides an improved matrix that not only stabilizes the active agent, in both liquid and in dried form (in terms of maintenance of chemical integrity and active agent potency) but also results in a microstructure array having good mechanical performance and good storage stability as well. Finally, as demonstrated by the exemplary formulations and microstructure arrays provided herein, in general, the combination can be effective to transdermally administer an active agent to achieve a therapeutic response that is at least equal to that obtained by intramuscular injection. The foregoing will now be described in greater detail in the sections which follow.

Microstructure Arrays

Microstructure Array Composition

General features of microstructure arrays suitable for use with the formulations and methods provided herein are described in detail in U.S. Patent Publication No. 2008/0269685, U.S. Patent Publication No. 2009/0155330, U.S. Patent Publication No. 2011/0006458, and U.S. Patent Publication No. 2011/0276028, the entire contents of which are explicitly incorporated herein by reference. Preferably, the microstructure array comprises an approximately planar base and attached to the base are a plurality of dissolving microstructures, each having an attachment point to the base and a distal tip to penetrate a subject's skin. See, e.g., **Fig. 1.**

Typically, at least at least a portion of the microstructures are formed of a biodegradable, bioerodible, bioabsorbable and/or biocompatible polymer matrix, preferably a biocompatible and water-soluble matrix. Biocompatible, biodegradable, bioabsorbable and/or bioerodible polymers suitable for use in the matrix include poly(lactic acid) (PLA), poly(glycolic acid) (PGA), poly(lactic acid-co-glycolic acid)s (PLGAs), polyanhydrides, polyorthoesters, polyetheresters, polycaprolactones (PCL), polyesteramides, poly(butyric acid), poly(valeric acid), polyvinylpyrrolidone (PVP), polyvinyl alcohol (PVA), polyethylene glycol (PEG), block copolymers of PEG-PLA, PEG-PLA-PEG, PLA-PEG-PLA, PEG-PLGA, PEG-PLGA-PEG, PLGA-PEG-PLGA, PEG-PCL, PEG-PCL-PEG, PCL-PEG-PCL, copolymers of ethylene glycol-propylene glycol-ethylene glycol (PEG-PPG-PEG, trade name of Pluronic® or Poloxamer®), dextran, hydroxyethyl starches such at hetastarch, tetrastarch or pentastarch, cellulose, hydroxypropyl cellulose (HPC), sodium carboxymethyl cellulose (Na CMC), thermosensitive HPMC (hydroxypropyl methyl cellulose), polyphosphazene, hydroxyethyl cellulose (HEC), other polysaccharides, polyalcohols, gelatin, alginate, chitosan, hyaluronic acid and its derivatives, collagen and its derivatives, polyurethanes, and copolymers and blends of these polymers.

Preferably, at least a portion of the microstructures comprises a biocompatible and water-soluble matrix comprising one or more hydrophilic, water-soluble polymers. In one or more embodiments, the entire portion of the microstructures comprises a biocompatible and water-soluble matrix. Preferred hydrophilic, water soluble polymers include polysaccharides, polyvinyl pyrrolidone, polyvinyl alcohol, polyethylene glycol, copolymers of ethylene glycol and propylene glycol (e.g., Pluronics®), block copolymers of PLGA and PEG, and the like. Particularly preferred polymers are polysaccharides. Polysaccharides preferred for use in the instant microstructure formulations are glucans, i.e., polysaccharides composed of D-glucose monomers linked by glycosidic bonds. The glucan may be an alpha-glucan, such as dextran, glycogen, pullulan, starch, and chemically modified versions thereof. Alternatively, the glucan may be a beta-glucan such as cellulose, curdlan, laminarin, chrysolaminarin, pleuran, zymosan, and the like, and chemically modified versions thereof, where water-soluble polysaccharides are particularly preferred. With respect to the foregoing, although any of a number of chemical modifications are possible, most typically, a chemically-modified polysaccharide is generally one that is hydroxyalkyl-modified or carboxy-methyl (CM) modified. Hydroxyalkyl-modified polysaccharides include those substituted with hydroxymethyl, hydroxyethyl, or hydroxypropyl groups, where the degree of substitution generally ranges from about 0.1 to about 0.8, or preferably from about 0.4 to 0.80. That is to say, the degree of substitution may be selected from about 0.1, 0.2, 0.3, 0.4, 0.5, 0.6, 0.7, and 0.8, or any range therebetween any two of the foregoing values. Preferred polysaccharides are dextran and chemically modified starch such as carboxymethylstarch and hydroxyalkylstarch (e.g., hydroxyethylstarch). Commercially available hydroxyethylstarch suitable for use in the instant formulations and microstructure arrays includes hetastarch (molar substitution of approximately 0.75) and tetrastarch (molar substitution of about 0.4). In one embodiment, the biocompatible and water-soluble matrix comprises as its only polymeric component, a polysaccharide as described above. In yet another embodiment, the biocompatible and water-soluble matrix comprises as its only polymeric component, a polysaccharide that is either dextran or a hydroxyethylstarch.

Generally, the biocompatible and water-soluble matrix comprises from about 35-80 weight percent polymer, e.g., polysaccharide, in the dried state, or from about 40 to 75 weight percent polymer, e.g., polysaccharide, or from about 45 to 70 weight percent polymer, e.g., polysaccharide. For example, the biocompatible and water-soluble matrix may comprise from about 35-80 weight percent of a polysaccharide (e.g., 35 wt%, 40 wt%, 45 wt%, 50 wt%, 55 wt%, 60 wt%, 65 wt%, 70 wt%, 75 wt%, or even 80 wt%, or a sub-range falling within any two of the foregoing values), in the dried state, or from about 40-75 weight percent polysaccharide, or from about 40 to 70 weight percent polysaccharide, where the polysaccharide is selected from dextran and chemically modified starch such as carboxymethylstarch and hydroxyalkylstarch (e.g., hydroxyethylstarch). In a related embodiment of the foregoing, the polysaccharide as described above is the only polymeric component of the biocompatible and water soluble matrix.

In the corresponding liquid formulations, i.e., for preparing the microstructure array layer, the weight percent polymer, e.g., polysaccharide, typically ranges from about 2-30 weight percent (e.g., 2 wt%, 3 wt%, 4 wt%, 5 wt%, 6 wt%, 7 wt%, 8 wt%, 9 wt%, 10 wt%, 11wt%, 12 wt%, 13 wt%, 14 wt%, 15 wt%, 16 wt%, 17 wt%, 18 wt%, 19 wt%, 20 wt%, 21 wt%, 22 wt%, 23 wt%, 24 wt%, 25 wt%, 26 wt%, 27 wt%, 28 wt%, 29 wt%, or 30 wt%, or a sub-range falling any two of the foregoing values), or preferably from about 3-20 weight percent, or even from 4-18 weight percent, depending upon the identities of the constituents in the liquid formulation.

Typically, the microprojection array includes one or more sugars, where the biodegradability or dissolvability of the microprojection array is facilitated by the inclusion of one or more sugars. Exemplary sugars include dextrose, fructose, galactose, maltose, maltulose, iso-maltulose, mannose, lactose, lactulose, sucrose, and trehalose. Preferred are sugar alcohols, for example lactitol, maltitol, sorbitol, mannitol, glycerol, xylitol, galactitol, and erythritol. Cyclodextrins can also be used advantageously in microneedle arrays, for example $\alpha$, $\beta$, and $\gamma$ cyclodextrins, for example hydroxypropyl-$\beta$-cyclodextrin and methyl-p-cyclodextrin. Particularly preferred are sugar alcohols, preferably acyclic polyhydric linear sugar alcohols, which, when combined with a polysaccharide as described above, appear to be particularly effective in both stabilizing the active agent components (e.g., nucleic acids, nucleotides, peptides and proteins or protein fragments) in the dried state, and for enhancing the mechanical properties of the microprojections by exhibiting a plasticizing-like effect on the polysaccharide polymer component. One particularly preferred sugar alcohol in this regard is sorbitol.

Generally, the biocompatible and water-soluble matrix comprises from about 20-60 weight percent sugar alcohol, e.g., linear sugar alcohol such as sorbitol, in the dried state, or preferably from about 25-50 weight percent sugar alcohol, or even from about 25-40 weight percent sugar alcohol. In the corresponding liquid formulations, i.e., for preparing the microstructure array layer, the weight percentage sugar alcohol ranges from about 0.5 to about 20 weight percent, preferably from about 1-15 weight percent, or even from about 1-12 weight percent.

The biodegradability of a microstructure array may also be facilitated by inclusion of water-swellable polymers such as crosslinked PVP, sodium starch glycolate, crosslinked polyacrylic acid, crosscarmellose sodium, celluloses, natural and synthetic gums, polysaccharides, or alginates.

In a multilayer array, the sugars and other polymers which facilitate biodegradability may, in certain embodiments, be located only in a layer or layers which encompass the microprojections. A preferred combination of components for the microprojection layer (i.e., plurality of microstructures) is that of a polysaccharide and sugar alcohol. Examples include dextran and a sugar alcohol such as sorbitol; or hydroxyethyl starch and a sugar alcohol such as sorbitol. In one or more embodiments, the plurality of microstructures comprise an active agent in a biocompatible and water-soluble matrix, where the biocompatible and water-soluble matrix comprises a polysaccharide and a sugar alcohol, where the polysaccharide is the only polymeric component and the sugar alcohol is the only sugar or modified sugar component (excluding any active agent that may be classified as a polymeric component or a sugar alcohol). However, the biocompatible and water-soluble matrix may comprise additional formulation additives as needed, such as one or more surfactants or chelating agents, or other additives. In certain instances, the inclusion of a surfactant may be advantageous for altering the surface tension and reducing the hydrophobic interactions of active agent in the liquid formulation. Generally, such additives are present in minor amounts in the biocompatible and water soluble matrix, e.g., at weight percentages in the solid formulations of less than about 20% by weight. Illustrative ranges for such additives in the solid formulations are from about 0.05% to about 20% by weight, or from about 0.5% to about 18%, or from about 0.05% to about 15% by weight, depending upon the nature of the additive and active agent components. Exemplary additives (to be described in greater detail below) include adjuvants, surfactants such as polysorbates such as polysorbate 20 and chelating agents such as EDTA.

The polymer(s) employed may possess a variety and range of molecular weights. The polymers employed are typically polydisperse, such that their molecular weights are actually weight average molecular weights. The polymers may, for example, have molecular weights of at least about 1 kilodalton, at least about 5 kilodaltons, at least about 10 kilodaltons, at least about 20 kilodaltons, at least about 30 kilodaltons, at least about 50 kilodaltons, or at least about 100 kilodaltons, or more. For biodegradable microstructures, it may be desirable to have biodegradable portion(s) comprising one or more polymers having a lower molecular weight, depending upon the selection of polymers. The strengthmolecular weight relationship in polymers is an inverse relationship, such that typically, polymers with lower molecular weights exhibit a lower strength and have a tendency to exhibit higher biodegradability and thus are more likely to break due to their lower mechanical strength. In one embodiment, at least the distal layer comprises at least one polymer having a lower molecular weight, e.g., less than about 100 kilodaltons. In another embodiment, at least the distal layer comprises a polymer having a molecular weight less than about 80 kilodaltons.

Exemplary formulations encompass those in which the biocompatible and water-soluble matrix of the dissolving microstructures comprises a polymer as described above having an average molecular weight falling within one of the following ranges: from about 1 -1,000 kDa, from about 5 - 800 kDa, or from about 15 - 700 kDa. For example, for polysaccharides such as dextran, illustrative average molecular weights include 1 kD, 40 kD, 60 kD, and 70 kD. For hydroxyethylstarch or HES, an illustrative average molecular weight is about 600,000 kD, where the molecular weight of the hydroxyethylstarch typically ranges from about 20 kD to about 2,500 kD. One exemplary molecular weight range for hydroxyethylstarch is from about 450 kD to about 800 kD. Illustrative polysaccharides for preparing the biocompatible and water-soluble matrix include dextran 40, dextran 60, dextran 70, tetrastarch and hetastarch.

The microstructure formulations provided herein are meant to encompass the formulations both in dried form, e.g., in the microstructures themselves, and in liquid form, e.g., for preparing the microstructures. Generally, the liquid formulations include components as described above in an aqueous solvent or a buffer. Exemplary buffers include phosphate buffered saline and histidine.

The distal layer (i.e., microstructure or microneedle layer) may comprise one or more polymers having a lower molecular weight while the proximal layer and/or the substrate or base may comprise polymers having a higher molecular weight. The polymers for the distal and/or proximal portions may be selected based at least partly on the molecular weight of the polymers to facilitate separation or detachment of at least a portion of the microstructures upon administration.

Generally, the number of microstructures forming the plurality in the array is at least about 50, preferably at least about 100, at least about 500, at least about 1000, at least about 1400, at least about 1600, or at least about 2000. For example, the number of microstructures in the array may range from about 1000 to about 4000, or from about 2000 to about 4000, or from about 2000 to about 3500, or from about 2200 to about 3200. The area density of microstructures, given their small size, may not be particularly high, but for example the number of microstructures per $cm^2$ may be at least about 50, at least about 250, at least about 500, at least about 750, at least about 1000, at least about 2000, or at least about 3000.

While the array itself may possess any of a number of shapes, the array is generally sized to possess a diameter of from about 5 millimeters to about 25 millimeters, or from about 7 to about 20 millimeters, or from about 8 to about 16 millimeters. Exemplary diameters include 5, 6, 7, 8, 9, 10, 11, 12, 13, 14, 15, 16, 17, 18, 19, 20, 21, 22, 23, 24, and 25 millimeters.

The sizes of the microneedles and other protrusions are a function of the manufacturing technology and of the precise application. In general, however, microstructures and other microprotrusions used in practice may be expected to have a height of at least about 20 to about 1000 microns, more preferably from about 50 to about 750 microns and most preferably from about 100 to about 500 microns. In specific but not limiting embodiments, the microstructures have a height of at least about 100 $\mu$m, at least about 150 $\mu$m, at least about 200 $\mu$m, at least about 250 $\mu$m, or at least about 300 $\mu$m. In general it is also preferred that the microprojections have a height of no more than about 1 mm, no more than about 500 $\mu$m, no more than about 300 $\mu$m, or in some cases no more than about 200 $\mu$m or 150 $\mu$m. Generally, the microprotrusions are long enough to penetrate at least partially through the stratum corneum layer of skin at some suitable point of application to a subject, e.g., a mammalian subject, for example the thigh, hip, arm, or torso. The microprojections may have an aspect ratio of at least 3: 1 (height to diameter at base), at least about 2:1, or at least about 1:1.

The microprojections may possess any suitable shape including, but not limited to polygonal or cylindrical. Particular embodiments include pyramidal including a four-sided pyramid, a funnel shape, a cylinder, a combination of funnel and cylinder shape having a funnel tip and a cylindrical base, and a cone with a polygonal bottom, for example hexagonal or rhombus-shaped. Other possible microprojection shapes are shown, for example, in U.S. Published Patent App. 2004/0087992. Microprojections may in some cases have a shape which becomes thicker towards the base, for example microprojections which have roughly the appearance of a funnel, or more generally where the diameter of the microprojection grows faster than linearly with distance to the microprojection distal end. It will be appreciate that polygonal microprojections may also have a shape which becomes thicker toward the base or where a radius or diameter grows faster than linearly with distance to the microprojection distal end. Where microprojections are thicker towards the base, a portion of the microprojection adjacent to the base, which may be called the "foundation," may be designed not to penetrate the skin.

In one or more embodiments, the microstructures have a sharp point or tip. A tip diameter of less than about 5 $\mu$m or 2 $\mu$m may be desirable. A tip diameter of less than about 1.5 $\mu$m is preferred, as is a tip diameter of less than

about 1 μm.

The microprojections are typically spaced about 0-500 μm apart. In specific, but not limiting embodiments, the microprojections are spaced about 0 μm, about 50 μm, about 100 μm, about 150 μm, about 200 μm, about 250 μm, about 300 μm, about 350 μm, about 400 μm, about 450 μm, or about 500 μm apart. The space between the microprojections may be measured from the base of the microprojections (base to base) or from the tip (tip to tip).

In further embodiments, at least a portion of the microprojections are detachable from the microprojection array. Detachable microprojection arrays are described in U.S. Patent Publication 2009/0155330 and in U.S. Patent Application No. 61/745,513, each of which is incorporated herein by reference. Detachable microprojection arrays may be accomplished by a number of approaches including, but not limited to, a layered approach in which the array is composed of multiple layers, and a layer comprising the areas where the microprojections attach to the base of the array is more readily degradable than other layers.

One advantage of detaching microprojections is the elimination of sharp disposal requirements, while another is elimination of needle stick injury. Additionally, detaching microprojections advantageously substantially reduce or eliminate misuse, for example, needle sharing, since the substrate or base absent the microprojections or with microprojections whose tips have been blunted due to biodegradability will not penetrate the skin. Another advantage of detaching microprojections is the avoidance of drug misuse, since the drug-enriched tips are dissolved in the skin, leaving no or minimal drug remaining in the array post-administration.

Alternatively, an array made of a homogeneous material may be employed, in which the material is more readily degradable at lower pH's. Arrays made of such a material will tend to degrade more readily near the attachment points because these, being closer to the surface of the skin, are at a lower pH than the distal ends of the microprojections. (The pH of the skin's surface is generally lower than that of the skin further inwards, pH being for example approximately 4.5 on the surface and approximately 6.5 to 7.5 inward).

Materials whose solubility is dependent on pH can be, for example, insoluble in pure water but dissolve in an acidic or basic pH environment. Using such materials or combination of materials, the arrays can be made to differentially biodegrade at the skin surface (pH approximately 4.5) or inside the skin. In the former embodiment, the whole array can biodegrade, while in the latter, the microprojection portion of the array will biodegrade allowing the base substrate to be removed and discarded. In a preferred embodiment, the microstructure array corresponds to the latter, wherein the microprojection portion of the array dissolves and biodegrades upon administration of active agent, allowing the base substrate to be removed and discarded.

Materials whose degradability in an aqueous medium is dependent on pH may be made, for example, by utilizing the acrylate copolymers sold by Rohm Pharma under the brand name Eudragit®, which are widely used in pharmaceutical formulations. A further example of a material with pH-dependent solubility is hydroxypropyl cellulose phthalate. Materials with pH-dependent solubility have been developed, for example, for use as enteric coatings in oral dosage forms. See, e.g., U.S. Patent No. 5,900,252 and Remington's Pharmaceutical Sciences (18th ed. 1990).

It may also be desirable, in certain instances, for the microprojection arrays provided herein to comprise one or more additional layers in addition to the biocompatible and water-soluble matrix layer which comprises a polymer such as a polysaccharide, a sugar alcohol, and the active agent. There are a number of reasons why arrays with multiple layers may be desirable. For example, it is often desirable that, compared to the whole volume of the microprojection array, the microprojections themselves possess a higher concentration of active ingredient such as an active agent. This is so, for example, because the microprojections can be expected, in many cases, to dissolve more rapidly, being in a more hydrated environment than the base of the array. Furthermore, in certain protocols for array application, the array may be left in for a short period of time during which essentially only the microprojections can dissolve to a substantial extent. The desirability of placing a higher concentration of active agent in the projections themselves is particularly acute when the active is costly. One way to achieve a higher concentration of active in the projections themselves is to have a first active-containing layer which includes the microprojections or a substantial proportion of the microprojections, and a second layer with a reduced or zero concentration of active which includes the base or a substantial proportion of the base.

Generally, in a preferred microstructure array configuration comprising two or more different layers, i.e., a layer comprising a plurality of microstructures or projections, and a base or backing layer supporting the microstructures, the base layer comprises a biocompatible, non-water soluble matrix. Once the microstructure array penetrates the skin, the microstructures dissolve, thereby delivering the active agent transdermally. The base layer preferably comprises any of a number of biocompatible, non-water soluble polymers including polyesters, polyaminoacids, polyanhydrides, polyorthoesters, polyurethanes, polycarbonates, polyetheresters, polycaprolactones (PCL), polyesteramides, and copolymers thereof. Illustrative polymers include polyacrylates, celluloses, poly(lactic acid) (PLA), poly(glycolic acid) (PGA), poly(lactic acid-co-glycolic acid)s (PLGAs), poly(butyric acid), poly(valeric acid). An exemplary backing or base layer comprises poly-lactide-poly-glycolide (PLGA75/25). See, e.g., Example 4.

Active Agents

As described previously, at least a portion of the dissolving microstructures provided herein comprises a biocompatible and water-soluble polymer matrix and an active agent.

The microstructures may comprise one or more active agents. In one or more embodiments, at least a portion of the microstructures may include a coating that may optionally contain one or more active agents.

In one embodiment, the active agent in the microprojection array is one or more proteins or peptides, for example, for use as a vaccine. These agents may include, for example, those approved in the United States for use against anthrax, diphtheria, hepatitis A, hepatitis B, *Haemophilus injluenzae* type b, human papillomavirus, influenza, Japanese encephalitis, Lyme disease, measles, meningococcal and pneumococcal diseases, mumps, pertussis, polio, rabies, rotavirus, rubella, shingles, smallpox, tetanus, tuberculosis, typhoid, varicella, and yellow fever. The active agent may comprise live attenuated or killed bacteria, live attenuated viruses, subunit vaccines, conjugate vaccines, synthetic vaccines, viral vectors, polysaccharide vaccines, and DNA vaccines. Among anthrax vaccines, particular preference is given to vaccines comprising the PA (protective antigen), particularly protective antigen which is recombinantly-produced (rPA, i.e., recombinant protective antigen). In another embodiment, the active agent is a hormone, such as parathyroid hormone (PTH), including the recombinant human parathyroid hormone (1-34).

Additional agents include those directed against avian (pandemic) influenza virus, *Campylobacter* sp., *Chlamydia* sp., *Clostridium botulinum, Clostridium difficile,* dengue fever virus, *E. coli,* Ebola virus, Epstein Barr virus, nontypeable *Haemophilus influenzae,* hepatitis C, hepatitis E, herpes viruses including herpes zoster, HIV, leishmanial and malarial parasites, meningococcal serogroup B, nicotine, parainfluenza, ragweed allergen, respiratory syncytial virus (RSV), Rift Valley fever virus, SARS-associated coronavirus, *Shigella* sp., *Staphylococcus aureus,* Streptococcus Group A (GAS), Streptococcus Group B (GBS), tick-borne encephalitis, Venezuelan equine encephalitis, and West Nile virus.

Due to the widespread use of vaccines, vaccine stability is an important consideration when there exists a choice between multiple types of vaccines for a particular condition. For example, in instances in which an active agent is heat sensitive, it is necessary to maintain a temperature-controlled supply chain for the vaccine, often referred to as a "cold chain." Cold chains for vaccines commonly target maintaining the vaccine at 2-8°C. This presents particular difficulties in poor countries with hot climates. Thus, for many vaccines, the solid-state formulation of the microprojection arrays provides enhanced stability and ease of handling over the corresponding liquid vaccines.

The microstructure array may also include additional excipients for inclusion in the biocompatible and water-soluble matrix, including, for example, adjuvants, preservatives, small molecule stabilizers, surfactants, and the like. Adjuvants include, for example, synthetic oliogodeoxynucleotides (ODNs) with or without and aluminum salts. Aluminum salts used as vaccine adjuvants include aluminum hydroxide, aluminum phosphate, and aluminum potassium sulfate, among others.

It is generally, but not always, desirable that the concentration of active agent by weight in the microprojection arrays is comparatively high, since it allows a higher concentration of active agent to be presented to the individual upon insertion of the microprojections into the skin. Illustrative concentrations in the solids forming the array (the biocompatible and water-soluble matrix) are as follows: at least about 0.1 %, 0.5%, 1%, 2%, 5%, 10%, 15% or 20% by weight active agent, e.g., vaccine. More preferably, the weight percent solids in the biocompatible and water-soluble matrix forming the microstructure projections ranges from about 1-15% active agent. That is to say, exemplary percentages by weight active agent, e.g., a vaccine, in the plurality of solid microprojections include 1%, 2%, 3%, 4%, 5%, 6%, 7%, 8%, 9%, 10%, 11%, 12%, 13%, 14%, and 15% or greater. For the corresponding liquid formulations, the amount of active agent will generally range from about 0.05 wt% to about 10 wt% active agent, or preferably, from about 0.5 wt% to about 5 wt% active agent.

The dose that is delivered to the body is that appropriate to elicit a substantial therapeutic and/or immune response in a large majority of individuals. In general, a desirable dose is at least about 0.1 ug/cm$^2$, at least about 0.5 ug/cm$^2$, at least about 1 ug/cm$^2$, at least about 2 ug/cm$^2$, at least about 5 ug/cm$^2$, or at least about 10 ug/cm$^2$.

Alternatively, the active agent dose may be measured as a percentage of the dose delivered by other paths, for example intramuscularly. It may be desirable, for example, to deliver at least about 1%, at least about 10%, at least about 25%, at least about 50%, at least about 75%, at least about 100%, at least about 150%, or at least about 200% of the dose delivered by other paths, for example of the dose delivered intramuscularly. Alternatively, it may be desired to deliver no more than about 200%, no more than about 150%, no more than about 100%, no more than about 75%, no more than about 50%, no more than about 25%, no more than about 10%, or no more than about 1% of the dose delivered by other paths. As with conventional transdermal patches, dose delivery (DDE) by a microprojection array may be less than the total active agent content of the microprojection arrays. See, e.g., Example 9.

Manufacturing the Microproiection Arrays

The microprojection arrays as provided herein can be fabricated by employing the techniques for the fabrication of two-layer arrays described in U.S. Provisional Patent Applications Nos. 60/1923,861 and 60/1925,462 (the priority documents for U.S. Patent Application No. 12/1148,180). Generally, a microstructure array as provided herein is prepared by (i) providing a liquid formulation comprising an active agent, a polysaccharide and a sugar alcohol in a buffer, (ii) dispensing the liquid formulation from (i) onto a mold having an array of microstructure cavities and filling the microstructure cavities to form a formulation-filled mold, (iii) drying the formulation-filled mold, (iv) placing a backing layer on the dried mold from (iii), whereby the backing layer forms a base having an attachment point to each of the microstructure cavities to provide a molded microstructure array, and (v) removing the microstructure array from (iv) from the mold.

In a particular embodiment of the method, the liquid formulation, which may be a solution or a suspension, comprises from about 3-20% by weight polysaccharide, from about 1-15% by weight sugar alcohol, and from about 0.05-5% by weight active agent, although particular amounts may, on occasion, vary. Illustrative formulations are provided in the accompanying examples and described briefly below. For example, a liquid formulation for preparing the desired microstructure array contains about 3-20 weight% polysaccharide component such as dextran or a hydroxyalkyl starch, from about 1-15% sugar alcohol such as sorbitol, and from about 0.05-5 weight percent active agent such as a vaccine. Alternatively, a liquid formulation for preparing the desired microstructure array contains about 5-15 weight% polysaccharide component such as dextran or a hydroxyalkyl starch, from about 3-12% sugar alcohol such as sorbitol, and from about 0.05-5 weight percent active agent such as a vaccine. The liquid formulations may optionally contain small amounts of additional formulation additives as needed, such as one or more surfactants or chelating agents, or other additives.

Example 1 provides illustrative liquid formulations containing an active agent, i.e., a vaccine. Illustrative formulations are as follows: (1) dextran, 14 wt%; sorbitol, 7 wt%, 0.6 % active; (2) dextran , 10.5 wt%; sorbitol, 5 wt%, 0.6% active; (3) dextran, 10.5 wt%; sorbitol, 5 wt%, 1.1% active; (4) dextran, 7 wt%; sorbitol, 3 wt%, 0.6% active; (5) dextran, 7wt%; sorbitol, 7 wt%, 0.6% active; (6) dextran, 14 wt%; sorbitol, 3 wt%, 0.6% active; (7) hydroxyethylstarch, 14 wt%; sorbitol, 7 wt%, 0.6% active; (8) hydroxyethylstarch, 10.5 wt%; sorbitol, 5 wt%, 0.6% active; (9) hydroxyethylstarch, 10.5 wt%; sorbitol, 5 wt%, 1.1% active; (10) hydroxyethylstarch, 7 wt%; sorbitol, 3 wt%, 0.6% active; (11) hydroxyethylstarch, 7 wt%; sorbitol, 7 wt%, 0.6% active; and (12) hydroxyethylstarch, 14 wt%; sorbitol, 3 wt%, 0.6% active. The bioactivity of the active agent is maintained in liquid formulations stored at either 5°C or 25°C, for a time period of 4 hrs, 1 day, 2 days, or 7 days. - i.e., a duration sufficient, at a minimum, to cover the microstructure array fabrication process. In considering maintenance/stability of active agent particle size, formulations having polysaccharide concentrations of less than about 14 wt% show good particle size stability under the conditions described.

Example 2 provides additional illustrative liquid compositions containing an active agent, i.e., a vaccine, an enhancer or adjuvant, or a combination of an active agent and enhancer, along with a polysaccharide selected from dextran and hydroxyethylstarch, and sorbitol, in phosphate buffered saline. Liquid formulations as described in detail in Table 2 contain from 7 to 17 weight percent dextran or hydroxyethylstarch, from 3 to 9 weight percent sorbitol, 0.75 weight percent active agent, and a small amount of an exemplary surfactant, polysorbate 20, at 0.02 weight percent. Illustrative liquid formulations in Table 3 contain from about 7 to 14 weight percent dextran or hydroxyethylstarch, and from about 3 to 9 weight percent sugar alcohol, sorbitol. Additional components include about 2.35 weight percent enhancer, a small amount, (0.02 weight percent) surfactant (polysorbate 20), and a small amount, 0.3 weight percent, of EDTA. Illustrative drug-in-tip liquid formulations as provided in Table 4 contain about 9 weight percent polysaccharide (dextran or hydroxyethyl starch), about 5 weight percent sorbitol, and 0.85 weight percent active agent. Additional formulation components include an enhancer and a small amount of surfactant, polysorbate 20. These representative liquid formulations are stable at 5°C for at least 48 hours, further illustrating the suitability of liquid formulations such as these for preparing a microstructure array.

Example 4 provides additional exemplary liquid formulations containing a combination of active agents, e.g., antigens. These liquid formulations comprise a combination of either dextran or hydroxyethylstarch, sorbitol, and active agent. A small amount of surfactant is also included in the formulations; the liquid formulations are stable at 5°C for at least 7 days and at 25°C for at least 2 days.

Turning back to the method of preparing a microstructure array, an array of microprotrusions or microprojections is generally formed by (a) providing a mold with cavities corresponding to the negative of the microprotrusions, (b) casting atop the mold a solution comprising components suitable for forming a biocompatible and water-soluble matrix, the active agent, and a solvent, (c) removing the solvent, (d) demolding the resulting array from the mold.

A microstructure array tool having different geometries can be used to make the negative mold (generally but not necessarily using polydimethylsilicone). Additional negative mold materials include polyurethanes, ceramic materials, waxes, and the like. This mold is then used to fabricate a microstructure array (MSA) which replicates the geometry of the original tool. One exemplary tool possesses a diamond shape with a microprojection height of 200 μm, a base width of 70 μm, and a projection-to-projection spacing of 200 μm.

Microstructure arrays containing an active agent can generally be prepared as follows. A liquid active agent formulation as described above, e.g., generally including a polysaccharide and a sugar alcohol, and optionally other excipients, adjuvants or additives, in an aqueous solvent or buffer, is introduced into the mold. The mold is then filled using any of a number of suitable techniques, such as compression, pressurization, and the like. After wiping, the liquid formulation contained in the mold is dried in either one or two primary drying steps, depending, for example, on the physicochemical properties of the respective liquid formulations, such as viscosity, solids content, surface interaction between liquid formulation and mold, etc. In one step primary drying, the liquid formulation contained in the mold is directly placed in an incubator oven at a temperature ranging from about 25°C to about 40°C to remove water. The one step drying can take place anywhere from 20 minutes to several hours. In a two-step drying process, the first step is a slow drying step in which the liquid formulation-filled mold is first placed in a controlled humidity chamber, e.g. with humidity at 75-90% RH, for about 1 min to 60 minutes at room temperature, followed by placement of the mold in an incubator oven at a temperature ranging from about 25°C to about 40°C for about 20 minutes to several hours.

Following drying, a backing layer is then cast on the dried formulation-containing mold to thereby attach to the plurality of microprojections. Preferably, the components of the microprojections (i.e., the components of the biocompatible and water soluble matrix) are not soluble in the solvent used in the backing layer. Generally, the solvent used in casting the backing layer is an organic solvent such as acetonitrile, ethanol, isopropyl alcohol, ethyl acetate, and the like. The backing layer is typically first dried in a compressed dry air (CDA) box for a period of time with controlled air flow, e.g., from about 15 minutes to 2 hours, followed by drying in a convection oven, e.g., at a temperature ranging from 35°C to 80°C, for about 30 - 90 minutes. A backing substrate is then optionally placed on the backing or base layer. The backing substrate material can be, e.g., a breathable nonwoven pressure sensitive adhesive or an ultraviolet-cured adhesive in a polycarbonate film, although many types of materials can be used.

Following removal from the mold, the microstructure array is typically die cut into appropriately sized sections, then may undergo a final drying step to remove residual moisture from the dried active agent-containing formulation and residual solvent from the backing layer. The final drying step may be conducted under vacuum (~0.05 torr) at room temperature or higher, e.g., 35°C, for an extended period of several hours.

If desired, the microstructure arrays can then be packaged or sealed, either collectively or individually, preferably in airtight packaging. The packaged microstructure array(s) may also include a dessicant. A microstructure array as provided herein may also be provided as part of a kit, where the kit may also include an applicator device.

The preparation of exemplary microstructure arrays in accordance with the disclosure is described in Examples 6, 7, and 8. Generally, the plurality of microstructures comprises from about 1 - 15 weight % (solids) active agent, from about 40-75 weight % (solids) polysaccharide, and from about 25-40 weight % (solids) sugar alcohol. More particularly, the plurality of microstructures may comprise from about 1 - 15 weight % (solids) active agent such as a vaccine, from about 40-75 weight % (solids) polysaccharide such as dextran or a hydroxyalkyl starch, and from about 25-40 weight % (solids) sugar alcohol such as sorbitol. Alternatively, the plurality of microstructures may comprise from about 2 - 12 weight % (solids) active agent such as a vaccine, from about 45-75 weight % (solids) polysaccharide such as dextran or a hydroxyalkyl starch, and from about 25-40 weight % (solids) sugar alcohol such as sorbitol. The active-agent comprising microsphere arrays described possess good mechanical performance, good active agent stability, as well as good performance based upon therapeutic response.

Characteristics of the Microstructure Arrays

The instant microstructure arrays comprise a dissolving biocompatible and water soluble matrix that stabilizes the active agent contained therein, in both liquid and in dried form (in terms of maintenance of chemical integrity and active agent potency) and additionally results in a microstructure array having good mechanical performance and good storage stability.

Exemplary microstructure arrays in accordance with the disclosure demonstrated advantageous active agent stability, both during manufacturing and upon storage. For instance, the active agent comprising biocompatible and water-soluble matrix, when dissolved in aqueous buffer at an active agent concentration ranging from about 0.1% to about 7% by weight, is further characterized by stability of the active agent for at least 7 days at 5°C, as measured by one or more of maintenance of active agent particle size, chemical integrity, and active agent potency. As shown in Examples 1, 2, and 3, liquid formulations used to prepare the microstructure array were sufficiently stable to maintain the integrity of the active agent during the manufacturing process. Moreover, exemplary dried microstructure arrays were found to possess good room temperature storage stability for an extended period of time (i.e., at least 3 months). See, e.g., Example 10. Finally, the immunogenic response resulting from the transdermal administration of an exemplary active agent via a microstructure array as provided herein was as least as good as the response observed for intramuscular administered liquid active agent (Example 11). Thus, the foregoing supports the advantageous features of the microstructure arrays, related formulations and methods provided herein.

Methods of Use

The methods, kits, microstructure arrays, and related devices and formulations described herein are used for transdermally administering an active agent to a human or veterinary subject.

The microstructure arrays described may be applied manually to the skin, e.g., by pressing the array into the skin. More preferably, an applicator is used to provide a mechanism for assisting in application of the microstructure array to and through the skin. A preferred type of applicator is one having a spring-loaded mechanism, to thereby drive the array into the skin by virtue of the energy stored in a spring. Suitable and illustrative applicators include those described in U.S. Publication No. 2011/0276027, which is incorporated herein in its entirety. For instance, an exemplary applicator will typically include a plunger or piston where the microstructure array is positioned on a distal end of the plunger, and an actuator (or actuating member) is actuated to thereby release the plunger. The plunger is typically held in a constrained or restrained position until released. Upon release of the plunger, the plunger then impacts the skin and thereby allows the microstructure array to pierce or rupture the skin surface. The remaining portion of the microstructure array may be removed from the plunger distal end automatically or manually.

Related aspects and embodiments

(1) In a first aspect, i.e., aspect 1, provided is a microstructure array comprising an approximately planar base and a plurality of biodegradable microstructures, each microstructure having an attachment point to the base and a distal tip to penetrate a subject's skin, wherein (i) the plurality of microstructures comprise an active agent in a biocompatible and water-soluble matrix, the biocompatible and water-soluble matrix comprising a hydrophilic polymer and a sugar alcohol, and (ii) the base comprises a biocompatible non-water soluble polymer matrix, wherein the microstructures, upon penetration of the subject's skin, undergo dissolution to thereby deliver the active agent.

(2) Embodiment 1. In a first embodiment related to aspect 1, provided is a microstructure array of aspect 1, wherein the polymer is selected from a polysaccharide, a modified cellulose, vinyl amide polymers, vinyl alcohol polymers, 1,2-epoxide polymers, or co-polymers thereof.

(3). Embodiment 2. In a second embodiment related to the first embodiment, the polysaccharide is a glucan or a chemically-modified glucan in the microstructure array.

(4). Embodiment 3. In a third embodiment, the polysaccharide is an alpha-glucan or a chemically modified alpha-glucan.

(5). Embodiment 4. In a fourth embodiment related to the first embodiment, the polysaccharide is a dextran or a chemically-modified starch in the microstructure array.

(6). Embodiment 5. In a fifth embodiment related to embodiment 3, the polysaccharide in the microstructure array is a chemically-modified starch selected from carboxymethyl starch and a hydroxyalkylstarch.

(7). Embodiment 6. In a sixth embodiment related to the microstructure array of embodiment 5, the hydroxyalkylstarch is hydroxyethylstarch (HES) or hydroxypropylstarch (HPS).

(8). Embodiment 7. In a seventh embodiment related to embodiments 5 and 6, the chemically-modified starch has a degree of substitution ranging from 0.80 to 0.40.

(9). Embodiment 8. In an eighth embodiment related to any one of the foregoing embodiments directed to a microstructure array, the sugar alcohol is selected from the group consisting of glycerol, xylitol, mannitol, sorbitol, galactitol, lactitol, erythritol, glycerol, maltitol, sucrose, and trehalose.

(10). Embodiment 9. In a ninth embodiment of the microstructure array of embodiment 1, the polysaccharide is dextran and the sugar alcohol is sorbitol.

(11). Embodiment 10. In a tenth embodiment of the microstructure array of embodiment 1, the polysaccharide is hydroxyethylstarch and the sugar alcohol is sorbitol.

(12). Embodiment 11. In an eleventh embodiment, provided is a microstructure array according to aspect 1 or any one of the foregoing embodiments, where the biocompatible and water-soluble matrix further comprises one or more excipients or adjuvants.

(13). Embodiment 12. In a twelfth embodiment related to embodiment 11, the one or more excipients comprises a surfactant.

(14). Embodiment 13. In a 13th embodiment related to embodiments 9 or 10, the active agent-comprising biocompatible and water-soluble matrix, when dissolved in aqueous buffer at an active agent concentration ranging from about 0.05% to about 20% by weight, is further characterized by stability of the active agent for at least 7 days at 5°C.

(15). Embodiment 14. In a 14th embodiment, provided is a microstructure array of aspect 1 or any one of embodiments 1-13 above, wherein the plurality of microstructures comprises from about 0.1 - 50 weight % (solids) active agent, about 20-95 weight % (solids) polysaccharide and about 5-50 weight % (solids) sugar alcohol.

(16). In a second aspect, provided is a liquid formulation suitable for forming a plurality of dissolving microstructures. The liquid formulation comprises an active agent, a polysaccharide and a sugar alcohol in a buffer, wherein the liquid formulation comprises from about 1-30% by weight polysaccharide, from about 1-30% by weight sugar alcohol, and from about 0.05-20% by weight active agent.

(17). In a first embodiment related to the second aspect, the polysaccharide is a dextran or a chemically-modified starch.

(18). In a second embodiment related to the second aspect, the polysaccharide is a chemically-modified starch that is either carboxymethyl starch or a hydroxyalkylstarch.

(19). In a third embodiment related to the second aspect, the hydroxyalkylstarch is either hydroxyethylstarch (HES) or hydroxypropylstarch (HPS).

(20). In a fourth embodiment related to the third embodiment of the second aspect, the hydroxyalkylstarch has a degree of substitution ranging from 0.80 to 0.40.

(21). In a fifth embodiment related to the second aspect, or any one of the first through fourth embodiments directed to the second aspect, the sugar alcohol is selected from the group consisting of glycerol, xylitol, mannitol, sorbitol, galactitol, lactitol, ertythritol, maltotritol, sucrose, and trehalose.

(22). In a sixth embodiment related to the second aspect, the polysaccharide is dextran and the sugar alcohol is sorbitol.

(23). In a seventh embodiment related to the second aspect, the polysaccharide is hydroxyethylstarch and the sugar alcohol is sorbitol.

(24). In an eighth embodiment related to the second aspect and any one of the foregoing embodiments related to the same, the liquid formulation comprises one or more excipients or adjuvants.

(25). In a ninth embodiment related to the eighth embodiment above, the one or more excipients comprises a surfactant.

26. In a tenth embodiment, provided is a liquid formulation according to the second aspect of any one of related embodiments 1-9, in a dried form.

(27). In a third aspect, provided is a method of making a microstructure array. The method comprises (i) providing a liquid formulation comprising an active agent, a hydrophilic polymer and a sugar alcohol in a buffer, wherein the liquid formulation comprises from about 1-30% by weight hydrophilic polymer, from about 5-50% by weight sugar alcohol, and from about 0.1-50% by weight active agent; (ii) dispensing the liquid formulation from (i) onto a mold having an array of microstructure cavities and filling the microstructure cavities to form a formulation-filled mold, (iii) drying the formulation-filled mold, (iv) placing a backing layer on the dried mold from (iii), whereby the backing layer forms a base having an attachment point to each of the microstructure cavities to provide a molded microstructure array, and (v) removing the microstructure array from (iv) from the mold.

(28). In a first embodiment related to the third aspect, the hydrophilic polymer is selected from a polysaccharide, a modified cellulose, vinyl amide polymers, vinyl alcohol polymers, 1,2-epoxide polymers, and co-polymers.

(29). In a second embodiment related to the third aspect, the method further comprises affixing the backing layer to a backing substrate.

(30). In a third embodiment related to the third aspect, the backing substrate is a breathable nonwoven pressure sensitive adhesive.

(31). In a fourth embodiment related to the third aspect, the backing substrate is an ultraviolet-cured adhesive in a polycarbonate film.

(32). In a fifth embodiment related to the third aspect, the backing substrate is formed from a polymer or metal.

(33). In a sixth embodiment related to the third aspect, or any one of the first through fifth embodiments above, following the dispensing step, excess liquid formulation is removed from the surface of the mold.

(34). In a seventh embodiment related to the third aspect, or any one of related embodiments 1 through 6, the liquid formulation is a solution or a suspension.

(35). In an eighth embodiment related to the third aspect, or any one of related embodiments 1 through 7, the microstructure cavities are filled by pressurization.

(36). In a ninth embodiment related to the third aspect, the liquid formulation is a liquid formulation according to the second aspect or any one of embodiments 1-9 related to the second aspect.

(37). In a fourth aspect, provided is a method of transdermally administering an active agent to a mammalian subject, comprising inserting into the skin of the subject a microstructure array according to the first aspect or any one of its related embodiments.

Examples

The following examples are illustrative in nature and are in no way intended to be limiting. Efforts have been made to ensure accuracy with respect to numbers (e.g., amounts, temperature, etc.) but some errors and deviations should be accounted for. Unless indicated otherwise, parts are parts by weight, temperature is in °C. and pressure is at or near atmospheric.

Abbreviations

| API | Active pharmaceutical ingredient, |
| HPLC | High performance liquid chromatography |
| MSA | Microstructure array |
| SDS-PAGE | Sodium dodecyl sulfate polyacrylamide gel electrophoresis |
| SEC | Size exclusion chromatography |
| SPE | Skin penetration efficiency |
| TDS | Transdermal delivery system |
| DSL | Dynamic Light Scattering |
| IM | Intramuscular |

EXAMPLE 1

LIQUID FORMULATIONS CONTAINING ACTIVE AGENT

An active agent stock solution containing a vaccine is added to a liquid solution containing a polysaccharide selected from Dextran 70 (pharmaceutical grade, MW 70,000) and hetastarch (hydroxyethyl starch, molar substitution approximately 0.75 (i.e., approximately 75 hydroxyethyl groups per 100 glucose units)) and sorbitol in histidine buffer and the resulting solution is gently mixed. Liquid formulations are stored at 5°C prior to use. Formulations are prepared as summarized in Table 1 below.

The stability of active agent in the liquid formulations is evaluated based upon particle size determined by dynamic light scattering and SDS-PAGE.

**Table 1**

| Liquid formulations Containing Active Agent | | | | | |
| --- | --- | --- | --- | --- | --- |
| Formulation Designation | Polymer | | Sugar | | Active Agent |
| | Type | Wt% | Type | Wt% | Wt% |
| A1 | Dextran 70 | 14 | Sorbitol | 7 | 0.6 |
| A2 | Dextran 70 | 10.5 | Sorbitol | 5 | 0.6 |
| A3 | Dextran 70 | 10.5 | Sorbitol | 5 | 1.1 |
| A4 | Dextran 70 | 7 | Sorbitol | 3 | 0.6 |
| A5 | Dextran 70 | 7 | Sorbitol | 7 | 0.6 |
| A6 | Dextran 70 | 14 | Sorbitol | 3 | 0.6 |
| A7 | Hetastarc h | 14 | Sorbitol | 7 | 0.6 |
| A8 | Hetastarc h | 10.5 | Sorbitol | 5 | 0.6 |
| A9 | Hetastarc h | 10.5 | Sorbitol | 5 | 1.1 |
| A10 | Hetastarc h | 7 | Sorbitol | 3 | 0.6 |
| A11 | Hetastarc h | 7 | Sorbitol | 7 | 0.6 |
| A12 | Hetastarc h | 14 | Sorbitol | 3 | 0.6 |

The active agent concentration in all liquid formulations is maintained at 5 mg/mL for this study. The liquid formulations are stored at 5°C or 25°C and the active agent in the formulations are analyzed at 4 hrs, 1, 2, and 7 days.

The SDS-PAGE results from similar formulations indicated no difference between the liquid formulations and neat active agent. No additional bands were observed - indicating that active agent is stable in the formulations.

The active agent particle size is stable in both the dextran and hydroxyethylstarch formulations having low (7%) polysaccharide concentrations. The active agent particle size is stable in all formulations stored at 5°C for up to 7 days or at 25°C for 4 hours. Formulations having medium polysaccharide concentrations (10.5 %) show good particle size stability at 5°C for 7 days and at 25°C for 4 hrs. The active agent particle size increases in the formulations having high polysaccharide concentrations (14%) stored at 25°C for 1 day or longer. The sorbitol component appears to have little effect on the active agent particle size.

The liquid formulations are stable at 5°C for at least 7 days and at 25°C for at least 4 hours - i.e., a duration sufficient, at a minimum, to cover the fabrication process. That is to say, the active agent integrity and its *in vitro* potency are maintained during the fabrication process, demonstrating the robustness of the process for preparing the microstructure arrays.

EXAMPLE 2

LIQUID FORMULATIONS CONTAINING A POLYSACCHARIDE

An active agent, an enhancer or adjuvant, or a combination of active agent and enhancer, is/are added to a liquid solution containing a polysaccharide selected from Dextran 70 (pharmaceutical grade, MW 70,000) and hetastarch (hydroxyethyl starch, molar substitution approximately 0.75 (i.e., approximately 75 hydroxyethyl groups per 100 glucose units)) and sorbitol in phosphate-buffered saline (pH 6.8), optionally containing additional additives/excipients such as polysorbate 20 (polyoxyethylene (20) sorbitan monolaurate) or ethylene diamine tetraacetic acid (EDTA). Formulations are prepared as summarized in Tables 2, 3 and 4 below. The stability of active agent in the liquid formulations is evaluated by SEC-HPLC. The stability of enhancer is evaluated by RP-HPLC.

The formulations containing active agent or enhancer are stable at 5°C for at least 48 hours.

**Table 2**

| Liquid Formulations Containing an Active Agent | | | | | | |
|---|---|---|---|---|---|---|
| **Formulation Designation** | **Polymer** | | **Sugar** | | **Active Agent** | **PS 20** |
| | **Type** | **Wt%** | **Type** | **Wt%** | **Wt%** | **Wt%** |
| B1 | Dextran 70 | 14 | Sorbitol | 9 | 0.75 | 0.02 |
| B2 | Dextran 70 | 14 | Sorbitol | 3 | 0.75 | 0.02 |
| B3 | Dextran 70 | 7 | Sorbitol | 9 | 0.75 | 0.02 |
| B4 | Dextran 70 | 7 | Sorbitol | 3 | 0.75 | 0.02 |
| B5 | Dextran 70 | 9 | Sorbitol | 5 | 0.85 | 0.02 |
| B6 | Hetastarch | 14 | Sorbitol | 9 | 0.75 | 0.02 |
| B7 | Hetastarch | 14 | Sorbitol | 3 | 0.75 | 0.02 |
| B8 | Hetastarch | 7 | Sorbitol | 9 | 0.75 | 0.02 |
| B9 | Hetastarch | 7 | Sorbitol | 3 | 0.75 | 0.02 |

**Table 3**

| Liquid Formulations Containing An Enhancer | | | | | | |
|---|---|---|---|---|---|---|
| **Formulation s** | **Polymer** | | **Sugar** | | **Enhanc er** | **PS 20** | **EDTA** |
| | **Type** | **Wt %** | **Type** | **Wt%** | **Wt%** | **Wt%** | **mg/mL** |
| C1 | Dextran 70 | 14 | Sorbitol | 9 | 2.35 | 0.02 | 0.3 |
| C2 | Dextran 70 | 14 | Sorbitol | 3 | 2.35 | 0.02 | 0.3 |
| C3 | Dextran 70 | 7 | Sorbitol | 9 | 2.35 | 0.02 | 0.3 |
| C4 | Dextran 70 | 7 | Sorbitol | 3 | 2.35 | 0.02 | 0.3 |
| C5 | Hetastarch | 14 | Sorbitol | 9 | 2.35 | 0.02 | 0.3 |
| C6 | Hetastarch | 14 | Sorbitol | 3 | 2.35 | 0.02 | 0.3 |
| C7 | Hetastarch | 7 | Sorbitol | 9 | 2.35 | 0.02 | 0.3 |

(continued)

| Liquid Formulations Containing An Enhancer | | | | | | | |
|---|---|---|---|---|---|---|---|
| **Formulation s** | **Polymer** | | **Sugar** | | **Enhanc er** | **PS 20** | **EDTA** |
| | **Type** | **Wt %** | **Type** | **Wt%** | **Wt%** | **Wt%** | **mg/mL** |
| C8 | Hetastarch | 7 | Sorbitol | 3 | 2.35 | 0.02 | 0.3 |

**Table 4**

| Examples of liquid DIT formulations with Active Agent/Enhancer Combination | | | | | | | | | **PS 20** |
|---|---|---|---|---|---|---|---|---|---|
| **Formulations** | **Polymer** | | **Sugar** | | | | | | |
| | **Type** | **Wt%** | **Type** | **Wt%** | **Type** | **Wt%** | **Type** | **Wt%** | **Wt%** |
| D1 | Dextran 70 | 9 | Sorbitol | 5 | Active agent | 0.85 | Enhancer | 2.25 | 0.02 |
| D2 | Hetastarch | 9 | Sorbitol | 5 | Active Agent | 0.85 | Enhancer | 2.25 | 0.02 |

EXAMPLE 3

LIQUID FORMULATIONS CONTAINING PLURALITY OF ACTIVE AGENT

Active agents are added to a liquid solution containing a polysaccharide selected from Dextran 70 (pharmaceutical grade, MW 70,000) and Hetastarch (hydroxyethyl starch, molar substitution approximately 0.75 (i.e., approximately 75 hydroxyethyl groups per 100 glucose units)), sorbitol, and the surfactant, polysorbate 20, in phosphate-buffered saline (pH 6.8). Formulations are prepared as summarized in Table 5 below.

The stability of the active agents in the liquid formulations are evaluated by a SRID (single radial immunodiffusion) assay. The liquid formulations are determined to be stable at 5°C for at least 7 days and at 25°C for at least 2 days.

**Table 5**

| Examples of Liquid Formulations | | | | | | | | | | | **PS 20** |
|---|---|---|---|---|---|---|---|---|---|---|---|
| **Formulation** | **Polymer** | | **Sugar** | | **Active Agents** | | | | | | |
| | **Type** | **Wt %** | **Type** | **Wt %** | **Type** | **Wt%** | **Type** | **Wt%** | **Type** | **Wt%** | **Wt %** |
| E1 | Dextran 70 | 11 | Sorbitol | 5.5 | Agent 1 | 0.48 | Agent 2 | 0.28 | Agent 3 | 0.28 | 2.2 |
| E2 | Dextran 70 | 7 | Sorbitol | 3 | Agent 1 | 0.48 | Agent 2 | 0.28 | Agent 3 | 0.28 | 2.2 |
| E3 | Dextran 70 | 11 | Sorbitol | 5.5 | Agent 1 | 0.48 | Agent 2 | 0.29 | Agent 3 | 0.29 | 1.8 |
| E4 | Hetastarch | 11 | Sorbitol | 5.5 | Agent 1 | 0.48 | Agent 2 | 0.28 | Agent 3 | 0.28 | 2.2 |
| E5 | Hetastarch | 7 | Sorbitol | 3 | Agent 1 | 0.48 | Agent 2 | 0.28 | Agent 3 | 0.28 | 2.2 |

EXAMPLE 4

BACKING LAYER: LIQUID FORMULATIONS

Illustrative polymer solutions were prepared for use in casting the backing layer of the microstructure array. The polymer solutions were prepared by dissolving the polymers in a solvent or solvent mixture at room temperature. Exemplary backing layer formulations are provided in Table 6.

**Table 6**

| Examples of Liquid Formulations for Backing Layer | | | | |
|---|---|---|---|---|
| **Formulations** | **Polymer** | | **Solvent** | |
| | **Type** | **Wt%** | **Type** | **Wt%** |
| F1 | PLGA (75/25) | 25 | Acetonitrile | 75 |
| F2 | PLGA (75/25) | 30 | Acetonitrile | 70 |
| F3 | PLGA (75/25) | 35 | Acetonitrile | 65 |

EXAMPLE 5

MICROSTRUCTURE ARRAY FABRICATION

<u>Backing substrate</u>

A backing substrate may be used to connect the backing layer with an applicator device. Exemplary backing substrates include (i) a breathable non-woven pressure sensitive adhesive which is placed on the top of backing layer and (ii) an UV-curable adhesive cast on the backing layer and cured by UV, among others.

<u>Tool</u>

A microstructure array tool with different geometries can be used to make the negative mold (generally using polydimethylsilicone). This mold is then used to fabricate a microstructure array (MSA) which replicates the geometry of the original tool. One exemplary tool used in these examples possesses a diamond shape with a microprojection height of 200 $\mu$m, a base width of 70 $\mu$m, and a projection-to-projection spacing of 200 $\mu$m.

<u>Fabrication</u>

Microstructure arrays containing an active agent as described herein are generally fabricated as set forth below. About 55 $\mu$l of liquid active agent formulation is introduced into the silicone mold, covered with an 18 X 18 mm glass cover slip, pressurized at 50 psi for 1 minute and wiped to remove excess formulation. Alternatively, about 75 $\mu$l of liquid formulation is cast into a silicone mold, covered with 22 X 30 mm glass cover slip, pressurized at 50 psi for 1 min, and wiped.

After wiping, the liquid formulation contained in the mold is dried in either one or two primary drying steps, depending, for example, on the physicochemical properties of the respective liquid formulations, such as viscosity, solids content, surface interaction between liquid formulation and mold, etc. In one step primary drying, the liquid formulation contained in the mold is directly placed in an incubator oven at 32°C for about 30 min to remove water. When two step drying was conducted, the first step is a slow drying step in which the liquid formulation-filled mold is first placed in a controlled humidity chamber with humidity at 85% RH, for 1 - 30 min at room temperature. The mold is then placed in an incubator oven at 32°C for about 30 min.

Following drying, a backing layer is then cast on the dried formulation-containing mold to thereby attach to the plurality of microprojections. The backing layer is first dried in a compressed dry air (CDA) box for 30 min with controlled air flow and then in a convection oven at 45°C for 30 - 90 min. A backing substrate is then placed on the backing layer.

Following removal from the mold, the microstructure array is die cut into either 11 mm (1 cm$^2$) or 14 mm (2 cm$^2$) sections, then undergoes the final drying step to remove residual moisture from the dried active agent-containing formulation and residual solvent from the backing layer. The final drying step is conducted under vacuum (~0.05 torr) at 35°C overnight. The microstructure arrays are sealed individually in Polyfoil pouches.

EXAMPLE 6

MICROSTRUCTURE ARRAY FABRICATION

To confirm that the active agent is not affected during drying, the liquid active agent -containing formulations are dried to thin solid films using the drying conditions which simulated the MSA fabrication process. The integrity of active agent in the solid films is characterized using SDS-PAGE and DLS. The bioactivity of active agent reconstituted from the solid film is assayed with ELISA.

There is no difference in the SDS-PAGE pattern among active agent in either liquid formulations or in solid films and the active agent standard. The active agent in both Formulation G1 and Formulation G2 is, therefore, not adversely affected by the drying process. Further, the results from the particle size evaluation and the *in vitro* potency assay all indicate that the drying process is well-tolerated by active agent formulations G1 and G2.

Active agent-containing microstructure arrays are fabricated as described below. Approximately 55 $\mu$l of liquid formulation A3 or A9 from Table 1 is cast into a silicone mold, covered with an 18 X 18 mm glass cover slip, pressurized at 50 psi for 1 min and wiped. Alternatively, about 75 $\mu$l of liquid formulation is cast into a silicone mold, covered with a 22 X 30 mm glass cover slip, pressurized at 50 psi for 1 min and wiped. After wiping, the liquid formulation contained in the mold is dried in two primary drying steps. The filled mold is placed in a controlled humidity chamber (85% RH) for 1 - 30 min at room temperature, followed by placement in an incubator oven at 32°C for about 30 min. A backing layer corresponding to formulation F2 is cast on the active agent-formulation containing mold to connect to the microprojections. The backing layer is first dried in a compressed dry air (CDA) box for 30 min with controlled air flow followed by drying in a convection oven at 45 °C for 30 - 90 min. A breathable non-woven pressure sensitive adhesive is then placed on the top of backing layer. The dried MSA is then removed from the mold and die cut into 1 or 2 $cm^2$ sizes. Further drying is conducted under vacuum (~0.05 torr) at 35°C overnight; the dried MSAs are sealed individually in Polyfoil pouches.

Active agent is extracted from the MSA in 5 mM histidine buffer, pH 6.8. Specifically, the active agent is extracted by submerging the MSA of 1 $cm^2$ area in 200 $\mu$L or 1.5 $cm^2$ area in 300 $\mu$L of 5 mM histidine buffer (pH 6.8) for approximately 30 min at room temperature on a low speed shaker. Active agent content in the liquid extracts is determined using a modified Lowry method. Content analysis of the MSA is carried out using SEC-HPLC.

The constituency of the dried compositions is summarized in Table 7.

**Table 7**

| Active Agent Dried MSA Formulations | | | | | |
|---|---|---|---|---|---|
| Formulation | Polymer | | Sugar | | Active Agent |
| | Type | Wt% | Type | Wt% | Wt% |
| G1 | Dextran 70 | 62.6 | Sorbitol | 30.3 | 7.1 |
| G2 | Hetastarc h | 62.6 | Sorbitol | 30.3 | 7.1 |

The MSA microprojections dissolve rapidly in excised pig skin. The active agent concentration in the microstructure array is measured by SEC-HPLC and is about 15-21 $\mu$g/$cm^2$.

Based upon these and the additional data described below, active agent containing MSAs comprising formulations as provided herein possess good mechanical performance, good active agent stability, as well as good performance based upon therapeutic response.

EXAMPLE 7

ACTIVE AGENT MICROSTRUCTURE ARRAY FABRICATION OPTIONALLY COMBINED WITH SECOND AGENT

Approximately 55 $\mu$l of liquid formulation B5 (active agent) or D1 (combination of active agent and second agent) as described in Tables 2 and 3 above is added to a silicone mold, covered with an 18 X 18 mm glass cover slip, pressurized at 50 psi for 1 min and wiped. Alternatively, about 75 $\mu$L of liquid formulation is cast into a silicone mold, covered with a 22 X 30 mm glass cover slip, pressurized at 50 psi for 1 min and wiped. After wiping, the liquid in mold formulation is dried in two primary drying steps. The mold containing the liquid formulation is placed in a controlled humidity chamber (85% RH) for 1 - 30 min at room temperature, followed by drying in an incubator oven at 32°C for about 30 min. A backing layer corresponding to backing Formulation F2 (Table 6), is cast on the active agent-formulation containing mold to connect to the microprojections. The backing layer is first dried in a compressed dry air (CDA) box with controlled air flow for 30 minutes, followed by drying in a convection oven at 45°C for 30 - 90 min. An UV adhesive is then placed on the top of the backing layer, covered with a 5 mil polycarbonate (PC) film to spread the adhesive, followed by curing using a UV Fusion system. The UV curing dose is 1.6 J/$cm^2$. After curing, the MSA comprising an active agent-containing a microprojection layer/a PLGA/UV adhesive backing layer on PC is removed from the mold and die cut into 1 - 2 $cm^2$ sections. The MSA then undergoes a final drying step to completely remove moisture from the microprojection layer and residual solvent from the backing layer. The final drying is conducted under vacuum (~0.05 torr) at 35°C overnight. The MSAs are sealed individually in Polyfoil pouches. Illustrative dried compositions of active agent or active agent, second agent combinations (i.e., those forming the solid MSA) are summarized in Table 8.

To determine active agent concentration in the final MSA, active agents are extracted from the MSA in 5 mM phosphate buffer, pH 8.0. The active agent in the MSA is measured by SEC-HPLC. Active agent/second agent load in the MSA is measured by UV for the second agent and the active agent load can be estimated based on the ratio of the two components in the formulation. The active agent in the MSA is about 16 $\mu$g/cm$^2$; second agent load in the MSA with the combined agents is about 55 $\mu$g/cm$^2$.

**Table 8**

| Exemplary Solid Compositions MSAx | | | | | | | | | |
|---|---|---|---|---|---|---|---|---|---|
| Formulation s | Polymer | | Sugar | | Active Agent | | | | PS2 0 |
| | Type | Wt% | Type | Wt % | Type | Wt% | Type | Wt % | Wt% |
| H1 | Dextran 70 | 60.9 | Sorbito I | 32. 9 | Agen t 1 | 6.1 | Agen t 2 | 0 | 0.14 |
| H2 | Dextran 70 | 52.9 | Sorbito I | 30. 4 | Agen t 1 | 5.4 | Agen t 2 | 13. 2 | 0.11 |

EXAMPLE 8

ACTIVE AGENT-CONTAINING MICROSTRUCTURE ARRAY FABRICATION

Approximately 55 $\mu$l of liquid formulation E1 or E3 as described in Table 4 above is added to a silicone mold, covered with an 18 X 18 mm glass cover slip, pressurized at 50 psi for 1 min and wiped. Alternatively, about 75 $\mu$L of liquid formulation is cast into a silicone mold, covered with a 22 X 30 mm glass cover slip, pressurized at 50 psi for 1 min and wiped. After wiping, the liquid in mold formulation is dried in two primary drying steps. The mold containing the liquid formulation is placed in a controlled humidity chamber (85% RH) for 1 - 30 min at room temperature, followed by drying in an incubator oven at 32 °C for about 30 min. A backing layer corresponding to backing Formulation F2 (Table 6), is cast on the active agent-formulation containing mold to connect to the microprojections. The backing layer is first dried in a compressed dry air (CDA) box with controlled air flow for 30 minutes, followed by drying in a convection oven at 45°C for 30 - 90 min. An UV adhesive is then placed on the top of the backing layer, covered with a 5 MIL polycarbonate (PC) film to spread the adhesive, followed by curing using a UV Fusion system. The UV curing dose is 1.6 J/cm$^2$. After curing, the MSA comprising an active-containing a microprojection layer/a PLGA/UV adhesive backing layer on PC is removed from the mold and die cut into 1 - 2 cm$^2$ sections. The MSA then undergoes a final drying step to completely remove moisture from the microprojection layer and residual solvent from the backing layer. The final drying is conducted under vacuum (~0.05 torr) at 35°C overnight. The MSAs are sealed individually in Polyfoil pouches. Illustrative dried formulations (i.e., those forming the solid MSA) are summarized in Table 9.

To determine active agent load in the final MSA, active agent is extracted from the MSA in phosphate buffer, pH 7.0. The agent load in the MSA is measured by SRID. Table 9 summarizes the concentrations for the MSAs.

**Table 9**

| Exemplary Solid Compositions | | | | | | | | | | | |
|---|---|---|---|---|---|---|---|---|---|---|---|
| Formulations | Polymer | | Sugar | | Active Agent | | | | | | PS 20 |
| | Type | Wt% | Type | Wt% | Type | Wt % | Type | Wt% | Type | Wt % | Wt% |
| I1 | Dextran 70 | 56.4 | Sorbit ol | 26.7 | Agen t 1 | 1.9 | Agen t 2 | 1.9 | Agen t 3 | 1.9 | 11.0 |
| I2 | Dextran 70 | 58.7 | Sorbit ol | 27.9 | Agen t 1 | 2.0 | Agen t 2 | 1.0 | Agen t 3 | 1.0 | 9.4 |

**Table 10**

| Active Agent Load in MSAs (SRID-determined) | | | | | |
|---|---|---|---|---|---|
| Formulation s | Active agent amount ($\mu$g/cm$^2$) | | | | |
| | Type | | Type | | Type | |
| I1 | Agen t 1 | 2.0 | Agen t 2 | 1.7 | Agen t 3 | 2.2 |
| I2 | Agen t 1 | 2.5 | Agen t 2 | 2.0 | Agen t 3 | 2.1 |

EXAMPLE 9

IN VITRO SKIN PENETRATION EFFICIENCY AND APPARENT DOSE DELIVERY EFFICIENCY

Full-thickness pig skin is excised from the abdomen and then clipped and shaved to remove hair bristles. The MSAs prepared as described above are applied to shaved skin sites using an applicator to apply a force suitable to insert at least a portion of each microprojection into the skin and held by hand *in situ* for a period time ranging from about 5-15 minutes. Application sites are dye stained and photographed to visualize the microstructure array penetrations. Penetrations are quantified using a computer-based image analysis program. Skin penetration efficiency (SPE) is then calculated based on the theoretical number of microstructures expected for the MSA as follows:

$$\% \; SPE = 100 \; x \; (\# \; Penetrations / \# \; Microstructures)$$

Residual active agent remaining in the MSA after the in vitro SPE tests is extracted by immersing the used MSA in an aqueous extraction medium for approximately 30 minutes, followed by analysis of the extract using a suitable analytical method, e.g. SEC-HPLC. The apparent delivered dose per unit and delivery efficiency are then calculated as follows:

$$Apparent \; delivered \; dose = Initial \; drug \; load - Residual \; drug$$

$$\% \; Drug \; delivery \; efficiency = 100 \; x \; Apparent \; delivered \; dose / Initial \; drug \; load$$

The SPE for MSAs as described in Example 6, formulation G1, is greater than 80%.

EXAMPLE 10

ACTIVE AGENT STABILITY IN THE MSA DURING FABRICATION AND UPON STORAGE

Active agent stability during fabrication of the MSA is monitored by taking samples during various fabrication steps and analyzing the active agent purity ("in-process stability"
For shelf life stability, the active agent-containing MSAs are stored at different storage conditions: e.g., 5°C, 25°C/65% RH and 40°C/75% RH. At a predetermined time points, the samples are analyzed for purity.
Different analytical methods are used to characterize the stability of the active agents employed: particle size, SDS-PAGE, SEC-HPLC and *in vitro* potency or SRID assay methods are used to monitor the stability of the active agent.
The MSAs containing the active agent in Formulation G1 are stable at 5° or 25°C for at least 3 months.

EXAMPLE 11

IN VIVO IMMUNOGENICITY STUDY OF ACTIVE AGENT TRANSDERMALLY ADMINISTERED USING MSAs

The *in vivo* immunogenicity of active agent administered by MSA is evaluated in pigs. All animals are primed with an IM injection of active agent. The active agent contained in a MSA is then used as a booster. The boost response of the MSA administered active agent is compared to the boost based upon an IM injection of active agent. A boost response from the active agent administered by MSA is at least as good as an IM liquid injection.
The *in vivo* immunogenicity of an active agent is evaluated in hairless guinea pigs. IM injection active agent/enhancer (adjuvant) liquid is used as control. All groups are administrated active agent three times: prime/boost/boost. Active agent delivered via MSA shows equal or better performance than IM injection. The quality of response, however, is much better with MSA administration than with IM.
While a number of exemplary aspects and embodiments have been discussed above, those of skill in the art will recognize certain modifications, permutations, additions and sub-combinations thereof. It is therefore intended that the following appended claims and claims hereafter introduced are interpreted to include all such modifications, permutations, additions and sub-combinations as are within their true spirit and scope.
All patents, patent applications, and publications mentioned herein are hereby incorporated by reference in their entireties. However, where a patent, patent application, or publication containing express definitions is incorporated by reference, those express definitions should be understood to apply to the incorporated patent, patent application, or publication in which they are found, and not necessarily to the text of this application, in particular the claims of this application, in which instance, the definitions provided herein are meant to supersede.

Various preferred features and embodiments of the present invention will now be described with reference to the following numbered paragraphs (paras).

1. A microstructure array comprising an approximately planar base and a plurality of biodegradable microstructures, each microstructure having an attachment point to the base and a distal tip to penetrate a subject's skin, wherein

(i) the plurality of microstructures comprises an active agent in a biocompatible and water-soluble matrix, the biocompatible and water-soluble matrix comprising a hydrophilic polymer and a sugar alcohol, and

(ii) the base comprises a biocompatible non-water soluble polymer matrix,

wherein the microstructures, upon penetration of the subject's skin, undergo dissolution to thereby deliver the active agent.

2. The microstructure array of para 1, wherein the polymer is selected from a polysaccharide, a modified cellulose, vinyl amide polymers, vinyl alcohol polymers, 1,2-epoxide polymers, or co-polymers thereof.

3. The microstructure array of para 2, wherein the polysaccharide is a glucan or a chemically-modified glucan.

4. The microstructure array of para 2, wherein the polysaccharide is a dextran or a chemically-modified starch.

5. The microstructure array of para 4, wherein the polysaccharide is a chemically-modified starch selected from carboxymethyl starch and a hydroxyalkylstarch.

6. The microstructure array of para 5, wherein the hydroxyalkylstarch is hydroxyethylstarch (HES) or hydroxypropylstarch (HPS).

7. The microstructure array of para 5 or 6, wherein the chemically-modified starch has a degree of substitution ranging from 0.80 to 0.40.

8. The microstructure array of any one of paras 1-7, wherein the sugar alcohol is selected from the group consisting of glycerol, xylitol, mannitol, sorbitol, galactitol, lactitol, erythritol, glycerol, maltitol, sucrose, and trehalose.

9. The microstructure array of para 4, wherein the polysaccharide is dextran and the sugar alcohol is sorbitol.

10. The microstructure array of para 6, wherein the polysaccharide is hydroxyethylstarch and the sugar alcohol is sorbitol.

11. The microstructure array of any one of paras 1-10, wherein the biocompatible and water-soluble matrix further comprises one or more excipients or adjuvants.

12. The microstructure array of para 9 or para 10, wherein the active agent-comprising biocompatible and water-soluble matrix, when dissolved in aqueous buffer at an active agent concentration ranging from about 0.05% to about 20% by weight, is further characterized by stability of the active agent for at least 7 days at 5°C.

13. The microstructure array of any one of paras 1-12, wherein the plurality of microstructures comprise from about 0.1 - 50 weight % (solids) active agent, about 20-95 weight % (solids) polysaccharide and about 5-50 weight % (solids) sugar alcohol.

14. A liquid formulation suitable for forming a plurality of dissolving microstructures, the liquid formulation comprising an active agent, a polysaccharide and a sugar alcohol in a buffer, wherein the liquid formulation comprises from about 1-30% by weight polysaccharide, from about 1-30% by weight sugar alcohol, and from about 0.05-20% by weight active agent.

15. The liquid formulation of para 14, wherein the polysaccharide is a dextran or a chemically-modified starch.

16. The liquid formulation of para 15, wherein the polysaccharide is a chemically-modified starch that is either carboxymethyl starch or a hydroxyalkylstarch.

17. The liquid formulation of para 16, wherein the hydroxyalkylstarch is either hydroxyethylstarch (HES) or hydroxypropylstarch (HPS).

18. The liquid formulation of para 17, wherein the hydroxyalkylstarch has a degree of substitution ranging from 0.80 to 0.40.

19. The liquid formulation of any one of paras 14-18, wherein the sugar alcohol is selected from the group consisting of glycerol, xylitol, mannitol, sorbitol, galactitol, lactitol, ertythritol, maltotritol, sucrose, and trehalose.

20. The liquid formulation of para 14, wherein (i) the polysaccharide is dextran and the sugar alcohol is sorbitol or (ii) the polysaccharide is hydroxyethylstarch and the sugar alcohol is sorbitol.

21. The liquid formulation of any one of paras 14-20, further comprising one or more excipients or adjuvants.

22. A dried form of the liquid formulation of any one of paras 14-21.

23. A method of making a microstructure array, comprising:

(i) providing a liquid formulation comprising an active agent, a hydrophilic polymer and a sugar alcohol in a buffer, wherein the liquid formulation comprises from about 1-30% by weight hydrophilic polymer, from about 5-50% by weight sugar alcohol, and from about 0.1-50% by weight active agent;
(ii) dispensing the liquid formulation from (i) onto a mold having an array of microstructure cavities and filling the microstructure cavities to form a formulation-filled mold,
(iii) drying the formulation-filled mold,
(iv) placing a backing layer on the dried mold from (iii), whereby the backing layer forms a base having an attachment point to each of the microstructure cavities to provide a molded microstructure array, and
(v) removing the microstructure array from (iv) from the mold.

24. The method of para 23, wherein the hydrophilic polymer is selected from a polysaccharide, a modified cellulose, vinyl amide polymers, vinyl alcohol polymers, 1,2-epoxide polymers, and co-polymers.
25. The method of para 23 or para 24, further comprising affixing the backing layer to a backing substrate.
26. The method of any one of paras 23-25, wherein following the dispensing step, excess liquid formulation is removed from the surface of the mold.
27. The method of any one of paras 23-26, wherein the liquid formulation is a solution or a suspension.
28. The method of any one of paras 23-27, wherein the microstructure cavities are filled by pressurization.
29. The method of any one of paras 23-28, wherein the liquid formulation is a liquid formulation according to any one of paras 14-21.
30. A method of transdermally administering an active agent to a mammalian subject, comprising inserting into the skin of the subject a microstructure array of any one of paras 1-13.

## Claims

1.  A microstructure array comprising an approximately planar base and a plurality of biodegradable microstructures, each microstructure having an attachment point to the base and a distal tip to penetrate a subject's skin, wherein

    (i) the plurality of microstructures comprises a biocompatible and water-soluble matrix, the water-soluble matrix comprising:

    > (a) 0.1-50 wt% (solids) of an active agent;
    > (b) 20-95 wt% (solids) of a polysaccharide; and
    > (c) 5-50 wt% (solids) of a sugar alcohol; and

    (ii) the base comprises a biocompatible non-water soluble polymer matrix.

2.  The microstructure array of claim 1, wherein the polysaccharide is selected from a glucan, a chemically-modified glucan, a dextran, or a chemically-modified starch, preferably wherein the chemically-modified starch is selected from carboxymethyl starch, and a hydroxyalkylstarch, more preferably wherein the hydroxyalkylstarch is hydroxyethylstarch (HES) or hydroxypropylstarch (HPS).

3.  The microstructure array of claim 2, wherein the polysaccharide is a chemically-modified starch selected from carboxymethyl starch and a hydroxyalkylstarch, preferably wherein the hydroxyalkylstarch is hydroxyethylstarch (HES) or hydroxypropylstarch (HPS), wherein the chemically-modified starch has a degree of substitution ranging from 0.80 to 0.40.

4.  The microstructure array of any one of claims 1 to 3, wherein the sugar alcohol is selected from the group consisting of glycerol, xylitol, mannitol, sorbitol, galactitol, lactitol, erythritol, glycerol, maltitol, sucrose, and trehalose.

5.  The microstructure array of claim 1, wherein the polysaccharide is dextran and the sugar alcohol is sorbitol, or wherein the polysaccharide is hydroxyethylstarch and the sugar alcohol is sorbitol.

6.  The microstructure array of any one of claims 1-5, wherein the biocompatible and water-soluble matrix further comprises one or more excipients or adjuvants.

7.  The microstructure array of any one of claims 1-5, wherein the biocompatible and water-soluble matrix further comprises at least one adjuvant selected from synthetic oliogodeoxynucleotides (ODNs), aluminum hydroxide, aluminum phosphate, and aluminum potassium sulfate.

8. The microstructure array of any one of claims 1-7, wherein the active agent is one or more proteins or peptides for use as a vaccine selected from agents for use against diphtheria, hepatitis A, hepatitis B, Haemophilus injluenzae type b, human papillomavirus, influenza, Japanese encephalitis, Lyme disease, measles, meningococcal disease, pneumococcal disease, mumps, pertussis, polio, rabies, rotavirus, rubella, shingles, smallpox, tetanus, tuberculosis, typhoid, varicella and yellow fever.

9. The microstructure array of any one of claims 1-7, wherein the active agent is one or more proteins or peptides for use as a vaccine selected from agents for use against avian (pandemic) influenza virus, Campylobacter sp., Chlamydia sp., Clostridium botulinum, Clostridium difficile, dengue fever virus, E. coli, Ebola virus, Epstein Barr virus, nontypeable Haemophilus influenzae, hepatitis C, hepatitis E, herpes viruses including herpes zoster, HIV, leishmanial and malarial parasites, meningococcal serogroup B, nicotine, parainfluenza, ragweed allergen, respiratory syncytial virus (RSV), Rift Valley fever virus, SARS-associated coronavirus, Shigella sp., Staphylococcus aureus, Streptococcus Group A (GAS), Streptococcus Group B (GBS), tick-borne encephalitis, Venezuelan equine encephalitis, and West Nile virus.

10. The microstructure array of any one of claims 1-9, wherein the biocompatible and water-soluble matrix comprises 0.1-20 wt% (solids) of the active agent

11. The microstructure array of any one of claims 1-9, wherein the biocompatible and water-soluble matrix comprises 1-15 wt% (solids) of the active agent

12. The microstructure array of any one of claims 1-11, wherein the biocompatible and water-soluble matrix comprises 40-75 wt% (solids) of the polysaccharide.

13. The microstructure array of any one of claims 1-12, wherein the biocompatible and water-soluble matrix comprises 25-40 wt% (solids) of the sugar alcohol.

14. The microstructure array of any one of claims 1-13 for use in transdermally administering the active agent to a mammalian subject and vaccinating the subject, wherein the active agent is one or more proteins or peptides.

15. The microstructure array for use according to claim 14, wherein at least a portion of the biodegradable microstructures dissolve after penetrating the subject's skin, thereby delivering the active agent.

Backing Layer

Drug loaded tips

Penetrated tips dissolve and release drug

skin

**FIG. 1**

Europäisches
Patentamt

European
Patent Office

Office européen
des brevets

# EUROPEAN SEARCH REPORT

Application Number

EP 22 15 6402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2008/269685 A1 (SINGH PARMINDER [US] ET AL) 30 October 2008 (2008-10-30)<br>* abstract *; figures 3,5c,6 *<br>* paragraphs [0008], [0031] – [0045], [0057] – [0059], [0062] – [0063], [0067], [0079], [0085], [0121] – [0123] * | 1-15 | INV.<br>A61B17/20<br>A61M37/00<br>A61K9/00 |
| X | WO 2012/122163 A1 (3M INNOVATIVE PROPERTIES CO [US]; ZHANG YING [US]; HANSEN KRISTEN J [U)<br>13 September 2012 (2012-09-13)<br>* abstract *; figures 1,3,4 *<br>* page 2, lines 12-15 *<br>* page 11, line 12 – page 12, line 14 *<br>* page 14, lines 12-14 *<br>* page 9, line 19 – page 10, line 2 *<br>* page 26, lines 1-5 * | 1-15 | |
| A | US 2011/121486 A1 (OH SEA-JIN [US] ET AL) 26 May 2011 (2011-05-26)<br>* abstract *; figures 1F,2E *<br>* paragraphs [0003], [0004], [0061] – [0062] * | 1-15 | TECHNICAL FIELDS SEARCHED (IPC)<br><br>A61B<br>A61M<br>A61K |
| A | US 2004/049150 A1 (DALTON COLIN CAVE [BE] ET AL) 11 March 2004 (2004-03-11)<br>* abstract *; figures 1,3 *<br>* paragraphs [0029] – [0031], [0036] – [0037] * | 1-15 | |

The present search report has been drawn up for all claims

| Place of search | Date of completion of the search | Examiner |
|---|---|---|
| The Hague | 5 July 2022 | Macaire, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 6402

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2022

| Patent document cited in search report | Publication date | Patent family member(s) | | Publication date |
|---|---|---|---|---|
| US 2008269685 A1 | 30-10-2008 | AU | 2008241470 A1 | 30-10-2008 |
| | | CA | 2686093 A1 | 30-10-2008 |
| | | EP | 2146689 A2 | 27-01-2010 |
| | | EP | 2664323 A1 | 20-11-2013 |
| | | ES | 2817249 T3 | 06-04-2021 |
| | | ES | 2820335 T3 | 20-04-2021 |
| | | US | 2008269685 A1 | 30-10-2008 |
| | | US | 2013292868 A1 | 07-11-2013 |
| | | US | 2015297878 A1 | 22-10-2015 |
| | | US | 2019184147 A1 | 20-06-2019 |
| | | WO | 2008130587 A2 | 30-10-2008 |
| WO 2012122163 A1 | 13-09-2012 | AU | 2012225609 A1 | 10-10-2013 |
| | | BR | 112013022955 A2 | 06-12-2016 |
| | | CA | 2829354 A1 | 13-09-2012 |
| | | CN | 103429222 A | 04-12-2013 |
| | | DK | 2683359 T3 | 20-07-2015 |
| | | EP | 2683359 A1 | 15-01-2014 |
| | | ES | 2542012 T3 | 29-07-2015 |
| | | JP | 5941072 B2 | 29-06-2016 |
| | | JP | 2014514022 A | 19-06-2014 |
| | | KR | 20140010425 A | 24-01-2014 |
| | | US | 2014066843 A1 | 06-03-2014 |
| | | US | 2018344631 A1 | 06-12-2018 |
| | | WO | 2012122163 A1 | 13-09-2012 |
| US 2011121486 A1 | 26-05-2011 | AU | 2009249610 A1 | 26-11-2009 |
| | | CA | 2728400 A1 | 26-11-2009 |
| | | CN | 102105108 A | 22-06-2011 |
| | | EP | 2296557 A1 | 23-03-2011 |
| | | ES | 2687258 T3 | 24-10-2018 |
| | | JP | 5584202 B2 | 03-09-2014 |
| | | JP | 2011520550 A | 21-07-2011 |
| | | KR | 20110025921 A | 14-03-2011 |
| | | US | 2011121486 A1 | 26-05-2011 |
| | | WO | 2009142741 A1 | 26-11-2009 |
| US 2004049150 A1 | 11-03-2004 | AT | 276788 T | 15-10-2004 |
| | | AU | 8395001 A | 05-02-2002 |
| | | CA | 2416869 A1 | 31-01-2002 |
| | | CA | 2657491 A1 | 31-01-2002 |
| | | CY | 1107870 T1 | 19-06-2013 |
| | | DE | 60105813 T2 | 17-11-2005 |
| | | DE | 60131688 T2 | 30-10-2008 |
| | | DK | 1301238 T3 | 10-01-2005 |
| | | DK | 1512429 T3 | 17-03-2008 |

EPO FORM P0459

For more details about this annex : see Official Journal of the European Patent Office, No. 12/82

**page 1 of 2**

## ANNEX TO THE EUROPEAN SEARCH REPORT
## ON EUROPEAN PATENT APPLICATION NO.

EP 22 15 6402

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2022

| Patent document cited in search report | Publication date | Patent family member(s) | | Publication date |
|---|---|---|---|---|
| | | EP | 1301238 A1 | 16-04-2003 |
| | | EP | 1512429 A1 | 09-03-2005 |
| | | ES | 2228937 T3 | 16-04-2005 |
| | | ES | 2295768 T3 | 16-04-2008 |
| | | JP | 4965053 B2 | 04-07-2012 |
| | | JP | 5595954 B2 | 24-09-2014 |
| | | JP | 2004504120 A | 12-02-2004 |
| | | JP | 2011156370 A | 18-08-2011 |
| | | PT | 1301238 E | 31-01-2005 |
| | | PT | 1512429 E | 18-02-2008 |
| | | US | 2004049150 A1 | 11-03-2004 |
| | | US | 2005197308 A1 | 08-09-2005 |
| | | US | 2014294919 A1 | 02-10-2014 |
| | | WO | 0207813 A1 | 31-01-2002 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61800543
- US 6451240 B
- US 6945952 B
- US 20020082543 A
- US 20050197308 A
- US 20090155330 A
- US 6219574 B
- US 6652478 B
- US 20080269685 A
- US 20110006458 A
- US 20110276028 A
- US 20040087992 A
- US 61745513
- US 5900252 A
- US 601923861 A
- US 601925462 B
- US 121148180 A
- US 20110276027 A

**Non-patent literature cited in the description**

- **JUNG-HWAN PARK et al.** Biodegradable polymer microneedles: Fabrication, mechanics, and transdermal drug delivery. *J. of Controlled Release,* 2005, vol. 104, 51-66
- **A.L. LEHNINGER.** Biochemistry. Worth Publishers, Inc,
- **J. MARCH.** Advanced Organic Chemistry. McGraw Hill
- Remington: The Science and Practice of Pharmacy
- **J. GRIFFITH HARDMAN ; L. L. LIMBIRD ; A. GILMAN.** Goodman & Gilman The Pharmacological Basis of Therapeutics
- Remington's Pharmaceutical Sciences. 1990